# EUROPEAN PATENT SPECIFICATION

(11) **EP 3 431 591 B1**
(45) Date of publication and mention of the grant of the patent: **16.09.2020**
(21) Application number: 17763398.9
(22) Date of filing: 09.03.2017
(51) Int. Cl.: C12N 15/00, A61K 31/7088, A61K 48/00, A61P 43/00, C12N 15/113

(54) **NUCLEIC ACID OLIGOMER FOR RNA HYBRID FORMATION**
NUKLEINSÄUREOLIGOMER FÜR RNA-HYBRIDFORMATION
OLIGOMÈRE D'ACIDE NUCLÉIQUE POUR LA FORMATION D'UN HYBRIDE D'ARN

(30) Priority: 09.03.2016 JP 2016046181
(43) Date of publication of application: 23.01.2019
(73) Proprietor: Tokyo University of Science Foundation, Tokyo 162-8601 (JP)
(72) Inventor: WADA, Takeshi, Tokyo 162-8601 (JP); YOSHINO, Reijiro, Tokyo 162-8601 (JP); HARA, Rintaro, Tokyo 162-8601 (JP); NUKAGA, Yohei, Tokyo 162-8601 (JP)
(74) Representative: Isarpatent
(86) International application number: PCT/JP2017/009566
(87) International publication number: WO 2017/155058

(56) References cited:
- JP-A- 2011 225 598
- JP-A- 2014 011 998
- JP-A- 2014 036 652
- JP-A- 2014 522 862
- JP-A- 2014 527 401
- JP-A- 2015 044 842
- OKA N ET AL: "Solid-Phase Synthesis of Stereoregular Oligodeoxyribonucleoside Phosphorothioates Using Bicyclic Oxazaphospholidine Derivatives as Monomer Units", JOURNAL OF THE AMERICAN CHEMICAL SOCIETY, AMERICAN CHEMICAL SOCIETY|, vol. 130, no. 47, 26 November 2008 (2008-11-26), pages 16031-16037, XP002597892, ISSN: 0002-7863, DOI: 10.1021/JA805780U [retrieved on 2008-11-04]
- WAGNER R W ET AL: "ANTISENSE GENE INHIBITION BY OLIGONUCLEOTIDES CONTAINING C-5 PROPYNE PYRIMIDINES", SCIENCE, AAAS, AMERICAN ASSOC. FOR THE ADVANCEMENT OF SCIENCE, US, vol. 260, no. 5113, 4 June 1993 (1993-06-04), pages 1510-1513, XP002038487, ISSN: 0036-8075, DOI: 10.1126/SCIENCE.7684856
- YOHEI NUKAGA ET AL: "Enhancement of the affinity of 2'-O-Me-oligonucleotides for complementary RNA by incorporating a stereoregulated boranophosphate backbone", RSC ADVANCES, vol. 5, no. 4, 28 November 2014 (2014-11-28), pages 2392-2395, XP055551902, DOI: 10.1039/C4RA11335G
- NATSUHISA OKA ET AL: "Stereocontrolled Synthesis of Oligoribonucleoside Phosphorothioates by an Oxazaphospholidine Approach", ORGANIC LETTERS, vol. 11, no. 4, 19 February 2009 (2009-02-19), pages 967-970, XP055179581, ISSN: 1523-7060, DOI: 10.1021/ol802910k
- PING LI ET AL: "Nucleoside and Oligonucleoside Boranophosphates: Chemistry and Properties", CHEMICAL REVIEWS, vol. 107, no. 11, 1 November 2007 (2007-11-01), pages 4746-4796, XP055551961, US ISSN: 0009-2665, DOI: 10.1021/cr050009p
- OKA N. ET AL.: 'Solid-Phase Synthesis of Stereoregular Oligodeoxyribonucleoside Phosphorothioates Using Bicyclic Oxazaphospholidine Derivatives as Monomer Units' J. AM. CHEM. SOC. vol. 130, no. 47, 2008, pages 16031 - 16037, XP002597892
- NUKAGA Y. ET AL.: 'Stereocontrolled Solid-Phase Synthesis of Phosphate/Phosphorothioate (PO/PS) Chimeric Oligodeoxyribonucleotides on an Automated Synthesizer Using an Oxazaphospholidine-Phosphoramidite Method' J. ORG. CHEM. vol. 81, no. 7, 03 March 2016, pages 2753 - 2762, XP055414136

### SEQUENCE LISTING

<110> Tokyo University of Science Foundation
<120> Nucleic acid oligomer
<130> CO-F04459-00
<150> JP 2016-046181
   <151> 2016-03-09
<160> 2
<210> 1
   <211> 12
   <212> RNA
   <213> Artificial
<220>
   <223> synthesized RNA oligonucleotide
<400> 1
   cgaaaaaaaa cg 12
<210> 2
   <211> 12
   <212> DNA
   <213> Artificial
<220>
   <223> synthesized DNA oligonucleotide
<400> 2
   cgaaaaaaaa cg 12

## Description

### Technical Field

The present invention relates to a nucleic acid oligomer for RNA hybrid formation.

### Background Art

An antisense molecule having a base sequence complementary to a target nucleic acid forms a double-strand complementary to the target nucleic acid, and can inhibit protein production from the target nucleic acid. In a case in which a disease-related gene is selected as a target nucleic acid, the antisense molecule acts directly on the disease-related gene, and therefore, is attracting attention as a medicine effective for gene therapy.

From the viewpoint of efficiently inhibiting the production of a protein to be targeted, an antisense molecule (nucleic acid oligomer) is required to have mainly cell membrane permeability, nuclease resistance, chemical stability in the body (for example, under an environment of pH 7.4), and a property of forming a stable double-strand only with a specific base sequence. As the antisense molecule, for example, a nucleic acid oligomer obtained by using a phosphorothioate compound (hereinafter referred to as "phosphorothioate type nucleic acid oligomer"), and a nucleic acid oligomer obtained by using a boranophosphate compound (hereinafter referred to as "boranophosphate type nucleic acid oligomer") are known, and there are many extensive research examples so far, and an antisense molecule has been put into practical use as a medicine. The phosphorothioate type nucleic acid oligomer has a bond of non-natural form, and therefore has high nuclease resistance and further high lipophilicity, so that there is an advantage that the cell membrane permeability can be expected, and further the immunoresponsiveness is low.

On the other hand, since the nucleic acid such as RNA, which is to be a target of an antisense molecule, is a chiral molecule, it is considered that the chirality of an antisense molecule influences the binding affinity to the complementary strand, that is, the double-strand forming ability, and indeed, it has been reported that the absolute steric configuration on a phosphorus atom of a phosphorothioate type nucleic acid oligomer influences the binding affinity to RNA. (for example, Nucleic Acids Res. 1995, Vol. 23, pp. 5000).

In addition, when a nucleic acid oligomer is administered, it is required to consider the interaction with many chiral molecules in a living body until the nucleic acid oligomer reaches a nucleic acid to be targeted. For this purpose, it is considered that the chirality of a nucleic acid oligomer to be administered is also required to be clarified.

In this regard, stereoselective synthesis of phosphorothioate type nucleic acid oligomers and boranophosphate type nucleic acid oligomers has been reported, and it has been reported that some of sterically highly controlled DNA oligomers and RNA oligomers have improved double-strand forming ability with a complementary strand (for example, International Publication WO 2011/108682, Japanese Patent Application Laid-Open (JP-A) No. 2015-093853, and Org. Lett. 2009, Vol. 11, pp. 967).
Oka et al. (J. Am. Chem. Soc. 2008, vol.130, no. 47, pages 16031-16037) describe that nucleoside 3'-*O*-bicylic oxazaphospholidine derivatives were designed as monomer units for a solid-phase synthesis of stereoregular oligodeoxyibonucleoside phosphorothioates (PS-ODNs).
Wagner et al. (For The Advancement of Science, US, 1993, vol. 260, no. 5113, pages 1510-1513) describe that nucleic acid oligomers comprising C-5 propynyl pyrimidines have enhanced affinity for complementary RNA.

### SUMMARY OF INVENTION

### Technical Problem

However, for example, a phosphorothioate type nucleic acid oligomer, which has been described in Org. Lett. 2009, Vol. 11, pp. 967, WO 2011/108682, and JP-A No. 2015-093853, improves the double-strand forming ability by controlling the stereo, however, it is difficult to say that the level is sufficient. Further, in these documents, only the structure related to the stereoselectivity in a nucleic acid oligomer has been focused, and other structures have been hardly taken into consideration. As described above, it can be said that there is still room for further improvement in the binding affinity (f double-strand orming ability) between an optically active nucleic acid oligomer and a complementary strand (RNA).

In view of the above circumstances, an object of the present invention is to provide a nucleic acid oligomer for RNA hybrid formation, which is excellent in the f double-strand forming ability with a complementary strand.

### Solution to Problem

Specific means for solving the above problems are as follows.
<1> A nucleic acid oligomer for RNA hybrid formation, including: a structure formed of from 3 to 50 continuous units of at least one unit of a nucleotide unit represented by the following General Formula (1) or a nucleotide unit represented by the following General Formula (2); and having a base length of from 8 bases to 50 bases, 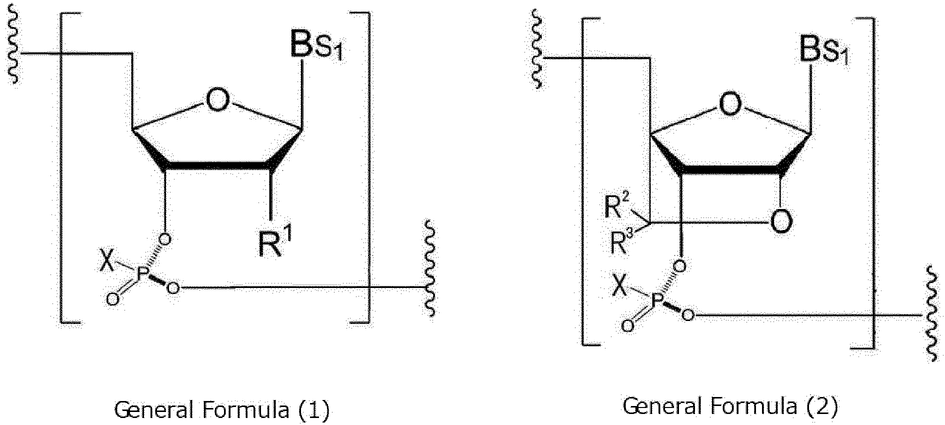 in which in General Formula (1), R¹ represents a hydrogen atom, an alkoxy group, an alkenyloxy group, an acyloxy group, a trialkylsilyloxy group, or a halogenyl group, and in General Formulae (1) and (2), each Bs₁ independently represents a pyrimidine base that may have a protecting group, a hydrogen atom bonded to a carbon atom at the 5-position of the pyrimidine base may be substituted with a group other than a hydrogen atom, in General Formulae (1) and (2), each X independently represents S⁻Z⁺ or BH₃⁻Z⁺, Z⁺ represents a counter cation, wherein when X is S⁻Z⁺, an absolute steric configuration of the phosphorus atom is Rp, and when X is BH3⁻Z⁺, an absolute steric configuration of the borano group is Sp, and wherein each R² and R³ independently represents a hydrogen atom, or an alkyl group having from 1 to 10 carbon atoms, and R² and R³ may be bonded to each other to form a ring and wherein, in the General Formulae (1) and (2), Bs₁ is a group represented by the following General Formula (3) or (4), 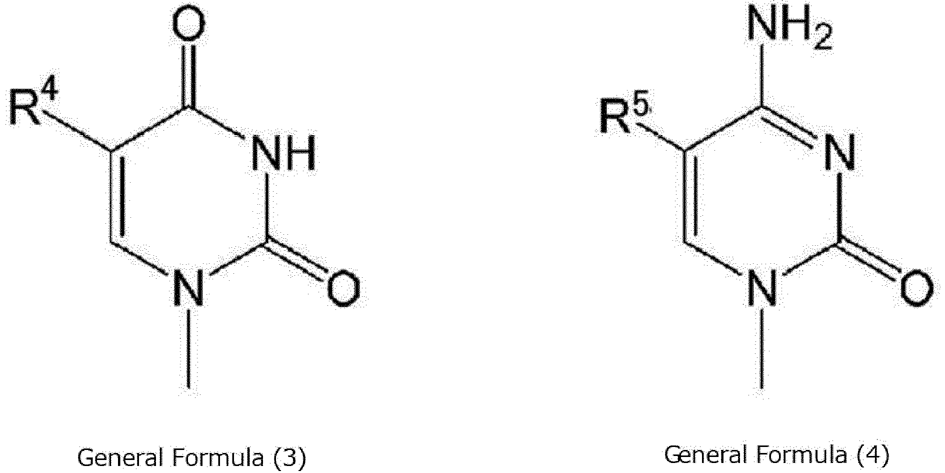 wherein, in General Formula (3), R⁴ represents a propynyl group, and each of a carbonyl group and an amino group in General Formula (3) may have a protecting group; and in General Formula (4), R⁵ represents a propynyl group, and each of a carbonyl group and an amino group in General Formula (4) may have a protecting group.
   Described and not claimed *per se* is that in General Formula (3), R⁴ represents a hydrogen atom, an alkyl group having from 1 to 10 carbon atoms, an alkenyl group having from 2 to 10 carbon atoms, an alkynyl group having from 2 to 10 carbon atoms, or a halogenyl group, and each of a carbonyl group and an amino group in General Formula (3) may have a protecting group, and in General Formula (4), R⁵ represents a hydrogen atom, an alkyl group having from 1 to 10 carbon atoms, an alkenyl group having from 2 to 10 carbon atoms, an alkynyl group having from 2 to 10 carbon atoms, or a halogenyl group, and each of a carbonyl group and an amino group in General Formula (4) may have a protecting group.
<2> The nucleic acid oligomer for RNA hybrid formation according to <1>, wherein the nucleic acid oligomer for RNA hybrid formation has a base length of from 10 bases to 30 bases.
<3> The nucleic acid oligomer for RNA hybrid formation according to <1> or <2>, in which the nucleic acid oligomer for RNA hybrid formation contains a structure formed of from 5 to 30 continuous units of at least one unit of the nucleotide unit represented by General Formula (1) or the nucleotide unit represented by General Formula (2).

### Advantageous Effects of Invention

According to the present invention, a nucleic acid oligomer for RNA hybrid formation, which is excellent in the double-strand forming ability with a complementary strand, can be provided.

### BRIEF DESCRIPTION OF DRAWINGS

Fig. 1A is a graph showing melting curves of double-strands of a nucleic acid oligomer in which the absolute steric configuration of a phosphorus atom in all of the phosphorothioate bonds is Sp with a natural form complementary strand RNA.
Fig. 1B is a graph showing melting curves of double-strands of a nucleic acid oligomer in which the absolute steric configuration of a phosphorus atom in all of the phosphorothioate bonds is Rp with a natural form complementary strand RNA.
Fig. 2A is a graph showing melting curves of double-strands of a nucleic acid oligomer in which the absolute steric configuration of a phosphorus atom in all of the phosphorothioate bonds is Sp with a natural form complementary strand DNA.
Fig. 2B is a graph showing melting curves of double-strands of a nucleic acid oligomer in which the absolute steric configuration of a phosphorus atom in all of the phosphorothioate bonds is Rp with a natural form complementary strand DNA.
Fig. 3A is a graph showing melting curves of double-strands of a nucleic acid oligomer d(ATA(Cs)6TAT), (Rp)-d(ATA(Cps)6TAT), or (Sp)-d(ATA(Cps)6TAT) with a natural form complementary strand RNA.
Fig. 3B is a graph showing melting curves of double-strands of a nucleic acid oligomer d(ATA(^{Me}Cs)6TAT), (Rp)-d(ATA(^{Me}Cps)6TAT), or (Sp)-d(ATA(^{Me}Cps)6TAT) with a natural form complementary strand RNA.
Fig. 3C is a graph showing melting curves of double-strands of a nucleic acid oligomer (Rp)-d(ATA(^{p}Cps)6TAT), or (Sp)-d(ATA(^{p}Cps)6TAT) with a natural form complementary strand RNA.

### DESCRIPTION OF EMBODIMENTS

The nucleic acid oligomer for RNA hybrid formation according to the present invention contains a structure formed of from 3 to 50 continuous units of at least one unit of a nucleotide unit represented by the following General Formula (1) or a nucleotide unit represented by the following General Formula (2), and the nucleic acid oligomer for RNA hybrid formation has a base length of from 8 to 50 bases.

The nucleic acid oligomer for RNA hybrid formation according to the present invention is characterized by having excellent double-strand forming ability with RNA that is a complementary strand.

Herein, the expression "double-strand forming ability with a complementary strand" is referred to as the easiness of forming a double-strand with RNA being a complementary strand. The double-strand forming ability can be expressed by, for example, a melting temperature (Tm) of a double-strand of the nucleic acid oligomer for RNA hybrid formation according to the present invention with a natural form complementary strand RNA. The value of Tm can be determined by, for example, obtaining a melting curve by measuring an absorbance in 260 nm at each temperature with the use of an ultraviolet and visible spectrophotometer equipped with a cell whose temperature can be variable.

The reason why the nucleic acid oligomer for RNA hybrid formation according to the present invention is excellent in the double-strand forming ability with a complementary strand is unknown, however, the inventors consider as follows. That is, in the case of forming a double-strand of RNA and DNA, or a double-strand of RNA and RNA, a double helical structure of A-form is formed. In this case, a sulfur atom or a borano group, which is bonded to a phosphorus atom in the nucleic acid oligomer for RNA hybrid formation according to the present invention, is brought into proximity with a group bonded to a carbon atom at the 5-position of a pyrimidine group in a nucleobase within the same molecule. At this time, in a case in which the absolute steric configuration of the phosphorus atom is one of Sp or Rp, for example, in the case of a sulfur atom, it is considered that in a case in which the absolute steric configuration is Rp, it is spatially easy to interact with the group at the 5-position of the pyrimidine group. Further in the case of a borano group, it is considered that in a case in which the absolute steric configuration is Sp, it is spatially easy to interact with the group at the 5-position of the pyrimidine group.

Therefore, it is considered that in a case in which the nucleobase is pyrimidine, by the generation of the strong interaction with the sulfur atom or borano group, the structure in the molecule is more stabilized, as a result of which the binding to the complementary strand is further strengthened, and as a result, the helical structure of A-form becomes more stabilized.

Therefore, it is considered that as the interaction described above is occursmore, as a result, the binding to a complementary strand (RNA) becomes stronger, for example, by allowing a structure formed of 3 or more continuous units of at least one unit of a nucleotide unit represented by the General Formula (1) and a nucleotide unit represented by the General Formula (2) to be present in a nucleic acid oligomer for RNA hybrid formation, the binding of the nucleic acid oligomer to the complementary strand becomes stronger regardless of whether the structural part is continuous or not, and the Tm value is further increased.

In the present specification, appropriately, the expression "hydrogen atom bonded to a carbon atom at the 5-position of a pyrimidine base" is simply referred to as "hydrogen atom at the 5-position of a pyrimidine base", and a group other than the hydrogen atom, which is substituted for the hydrogen atom, may be referred to as "substituent at the 5-position of a pyrimidine base".

In the present specification, the expression "monomer" is referred to as a nucleic acid that is used to introduce a nucleotide unit of a nucleic acid oligomer when a nucleic acid oligomer is produced.

The nucleotide unit represented by General Formula (1) and the nucleotide unit represented by General Formula (2) each may be referred to as "specific nucleotide unit", and a nucleotide unit other than the specific nucleotide unit may be simply referred to as "another nucleotide unit". In addition, in a nucleic acid oligomer for RNA hybrid formation, a structure of a portion of from 3 to 50 specific nucleotide continuous units may also be referred to as "specific nucleotide structure".

An absolute steric configuration of a phosphorus atom of the nucleic acid oligomer for RNA hybrid formation can be determined, for example, by an enzymatic hydrolysis method and ³¹P-nuclear magnetic resonance spectrum (³¹P-NMR) after purification of the synthesized oligomer by HPLC or the like. Further, a stereochemical purity (diastereomeric purity) in a nucleic acid oligomer for RNA hybrid formation can be obtained by the measurement using the ³¹P-nuclear magnetic resonance spectrum, a polarimeter, or the like.

In the present specification, the term "process" is not limited to an independent process, and even in the case of a process that cannot be clearly distinguished from other processes, the process is included in the term as long as the expected purpose of the process can be achieved.

In addition, in the present specification, the expression "to" is intended to indicate a range including the numerical values described before and after the "to" as a minimum value and a maximum value, respectively.

Further, in the present specification, in a case in which multiple substances corresponding to each component are present in a composition, the amount of each component in the composition means the total amount of respective amounts of the multiple substances present in the composition, unless otherwise specified.

Hereinafter, the present invention will be described.

### <Nucleic Acid Oligomer for RNA Hybrid Formation>

The nucleic acid oligomer for RNA hybrid formation contains a structure formed of from 3 to 50 continuous units of at least one unit of a nucleotide unit represented by the following General Formula (1) and a nucleotide unit represented by the following General Formula (2), and has a base length of from 8 bases to 50 bases.

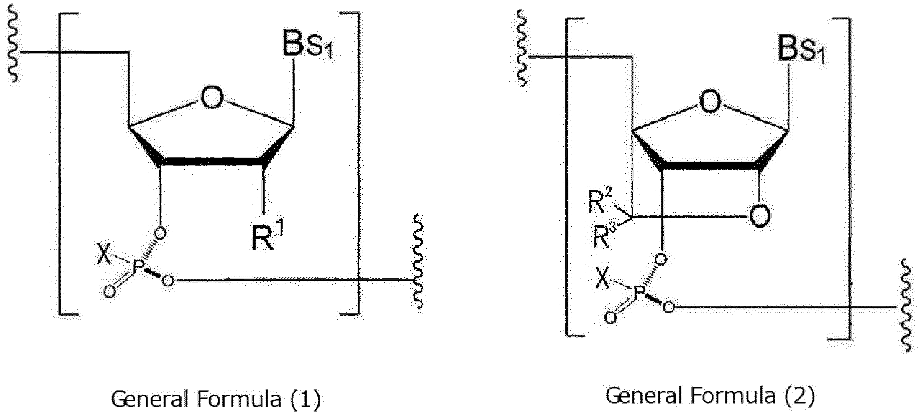

In General Formula (1), R¹ represents a hydrogen atom, an alkoxy group, an alkenyloxy group, an acyloxy group, a trialkylsilyloxy group, or a halogenyl group. In General Formulae (1) and (2), each Bs₁ independently represents a pyrimidine base that may have a protecting group, and a hydrogen atom bonded to a carbon atom at the 5-position of a pyrimidine base may be replaced with a group other than a hydrogen atom. In General Formulae (1) and (2), each X independently represents S⁻Z⁺ or BH3⁻Z⁺, Z⁺ represents a counter cation, wherein when X is S⁻Z⁺, an absolute steric configuration of the phosphorus atom is Rp, and when X is BH3⁻Z⁺, an absolute steric configuration of the borano group is Sp, and wherein. In General Formula (2), each R² and R³ independently represents a hydrogen atom, or an alkyl group having from 1 to 10 carbon atoms. R² and R³ may be bonded to each other to form a ring and wherein, in General Formulae (1) and (2), Bs₁ is a group represented by the following General Formulae (3) or (4): 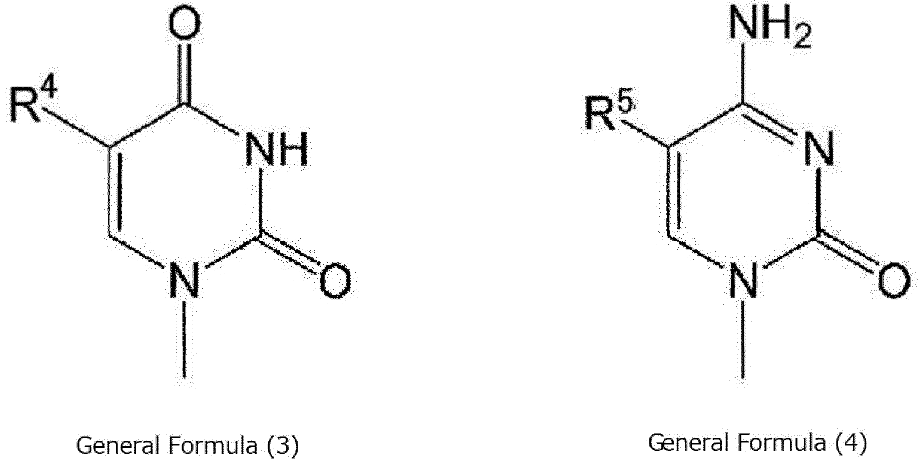 wherein, in General Formula (3), R⁴ represents a propynyl group, and each of a carbonyl group and an amino group in General Formula (3) may have a protecting group; and in General Formula (4), R⁵ represents a propynyl group, and each of a carbonyl group and an amino group in General Formula (4) may have a protecting group.

The length (base length) of the nucleic acid oligomer for RNA hybrid formation may be from 8 bases to 50 bases, and a desired length may be appropriately selected depending on the kind, length, or the like of the nucleic acid (RNA) to be targeted. The length of the nucleic acid oligomer for RNA hybrid formation according to the present invention is not particularly limited, however, from the viewpoint of the application to an antisense medicine, the length is preferably from 10 to 30 bases, and more preferably from 10 to 21 bases.

From the viewpoint of the double-strand forming ability, the base sequence of the nucleic acid oligomer for RNA hybrid formation is appropriately selected so as to be a base sequence complementary to the base sequence of RNA to be targeted, but may be a base sequence containing one or more different bases in some cases.

In General Formulae (1) and (2), each X may be S⁻Z⁺, or BH₃⁻Z⁺. As the Z⁺, for example, a cation derived from an organic amine compound, or inorganic and metal cations can be exemplifid. Examples of the cation derived from an organic amine compound include a tertiary alkyl ammonium ion, a heteroaromatic iminium ion, and a heterocycle iminium ion. Examples of the inorganic and metal cations include an ammonium ion, and a monovalent metal ion. Specific examples of the Z⁺ include triethylammonium ion, N,N-diisopropylethylammonium ion, pyridinium ion, 1,8-diazabicyclo[5,4,0]undeca-7-enium ion, ammonium ion, lithium ion, sodium ion, and potassium ion.

In the nucleic acid oligomer for RNA hybrid formation, 5'-end may be a hydroxyl group, or a protecting group of a hydroxyl group, and 3'-end may be a hydrogen atom, or a protecting group of a hydroxyl group. Examples of the protecting group of a hydroxyl group include an appropriate protecting group, for example, an acetyl protecting group such as acetyl group or a phenoxyacetyl group (Pac); a benzyl protecting group such as a benzyl group or a 4-methoxybenzyl group; a trityl protecting group such as a benzoyl group, a pivaloyl group or a 4,4'-dimethoxytrityl group (DMTr); a silyl protecting group sucu as a trimethylsilyl group (TMS) or a tert-butyldimethylsilyl group (TBDMS); and an ether protecting group such as a 2-(cyanoethoxy) ethyl group (CEE) or a cyanoethoxymethyl group (CEM) can be used. With regard to the protecting group of a hydroxyl group, reference can be made to a book such as "Protective Groups in Organic Synthesis" by Green, et al., 3rd Edition, 1999, John Wiley & Sons, Inc. In a case in which 5'-end and/or 3'-end in the nucleic acid oligomer for RNA hybrid formation is a protecting group, it is preferred that the protecting group is a protecting group different from other protecting groups so that the protecting group or other protecting groups can be selectively eliminated in a synthesis process. As the protecting group at 5'-end and/or 3'-end in the nucleic acid oligomer for RNA hybrid formation, for example, a trityl protecting group is preferably used, and a 4,4'-dimethoxytrityl group is more preferably used.

The specific nucleotide structure in the nucleic acid oligomer for RNA hybrid formation may be a structure formed of from 3 to 50 continuous units of at least any one unit of a nucleotide unit represented by General Formula (1) and a nucleotide unit represented by General Formula (2), and for example, the specific nucleotide structure may be a structure formed of only the nucleotide unit represented by General Formula (1), or may be a nucleic acid oligomer formed of only the nucleotide unit represented by General Formula (2). Further, the specific nucleotide structure may also be a structure formed of continuous constant repeat patterns of the nucleotide unit represented by General Formula (1) and the nucleotide unit represented by General Formula (2) or a structure formed of disorderly continuous units of the nucleotide unit represented by General Formula (1) and the nucleotide unit represented by General Formula (2).

In addition, the nucleic acid oligomer for RNA hybrid formation according to the present invention may be a nucleic acid oligomer having other nucleotide units (hereinafter appropriately referred to as "chimeric oligomer") as long as the nucleic acid oligomer includes the specific nucleotide unit having a structure formed of from 3 to 50 continuous units. In the case of a chimeric oligomer, there is no particular limitation on the other nucleotide units, and as the other nucleotide units, nucleotide units obtained by condensing a natural form of the monomer described in (V) of paragraph 0051 of JP-A No. 2015-093853 and a derived natural form of a monomer, or the like can be exemplifid.

In the nucleic acid oligomer for RNA hybrid formation, it is preferred that the specific nucleotide structure contains a structure formed of from 5 to 30 continuous units regardless of whether or not the oligomer is a chimeric oligomer. When the specific nucleotide structure contains 5 or more units, the double-strand forming ability of the nucleic acid oligomer for RNA hybrid formation with RNA can be further increased, and when the specific nucleotide structure contains 30 or less units, the usability is further improved. Further, it is more preferred that the specific nucleotide structure contains from 7 to 25 continuous units.

In the case of a chimeric oligomer, with regard to the position of the specific nucleotide structure in the chimeric oligomer in the nucleic acid oligomer for RNA hybrid formation, there is no particular limitation, and for example, the specific nucleotide structure may be present on 5'-end side or 3'-end side, or may be present continuously in a chimeric oligomer. In addition, in the chimeric oligomer, the total number of the nucleotide units constituting each of the specific nucleotide structures may be simply referred to as "total sum of specific nucleotide units".

In the nucleic acid oligomer for RNA hybrid formation, the ratio of the total sum of the specific nucleotide units to the base length of the nucleic acid oligomer is not particularly limited, and is preferably 40% or more from the viewpoint of the double-strand forming ability. When the ratio is 40% or more, it is easier to improve the Tm value. Further, the ratio is more preferably 60% or more, and particularly preferably 70% or more.

In R¹ in General Formulae (1) and (2), examples of the alkoxy group include a methoxy group, an ethoxy group, a n-propoxy group, an i-propoxy group, a n-butoxy group, a sec-butoxy group, a tert-butoxy group, and a n-pentyloxy group.

Examples of the alkenyloxy group include a vinyloxy group, an allyloxy group, a 1-propenyloxy group, an isopropenyloxy group, a 2-methyl-1-propenyloxy group, a 2-methylallyloxy group, and a 2-butenyloxy group.

Examples of the acyloxy group include an alkyl-carbonyloxy group having from 1 to 6 carbon atoms, (for example, a methylcarbonyloxy group, or an ethylcarbonyloxy group), and aryl-carbonyloxy having from 6 to 10 carbon atoms (for example, a benzoyloxy group).

Examples of the trialkylsilyloxy group include a trimethylsilyloxy group, and a triethylsilyloxy group.

Examples of the halogenyl group include a fluoro group, a chloro group, and a bromo group.

As the R¹, from the viewpoint of the double-strand forming ability, in particular, a hydrogen atom, an alkoxy group, and a trialkylsilyloxy group are preferred.

In addition, as the alkoxy group, an alkoxy group having from 1 to 12 carbon atoms is preferred, and an alkoxy group having from 1 to 6 carbon atoms is more preferred.

Further, as the halogenyl group, a fluoro group is more preferred.

In the General Formulae (1) and (2), each Bs₁ represents a pyrimidine base, and may have a protecting group. In a case in which the pyrimidine base has a protecting group, the kind of the protecting group is not particularly limited. In a case in which a cytosine base having an amino group is selected as the pyrimidine base, from the viewpoint of protecting the amino group of the cytosine base, examples of the protecting group include a benzyl group, a 4-methoxybenzoyl group, an acetyl group, a propionyl group, a butyryl group, an isobutyryl group, a phenylacetyl group, a 4-tert-butylphenoxyacetyl group, a 4-isopropylphenoxyacetyl group, and a (dimethylamino)methylene group.

In addition, the hydrogen atom bonded to a carbon atom at the 5-position of a pyrimidine base may be replaced with an atom other than a hydrogen atom. The substituent that may be used for the replacement is not particularly limited. However, from the viewpoint of the double-strand forming ability of the nucleic acid oligomer for RNA hybrid formation according to the present invention with RNA, the substituent is preferably any one group selected from an alkyl group, a cycloalkyl group, an alkenyl group, an aryl group, a heteroaryl group, an alkynyl group, an acyl group, and a halogenyl group. Further, the substituents to be bonded to a carbon atoms at the 5-positions of each of the pyrimidine bases in the nucleic acid oligomer may be the same as or different from each other.

As the alkyl group, for example, a linear or branched alkyl group having from 1 to 10 carbon atoms can be exemplifid. Specific examples of the alkyl group include a methyl group, an ethyl group, a n-propyl group, an i-propyl group, a n-butyl group, a t-butyl group, a n-hexyl group, and a n-octyl group.

Examples of the cycloalkyl group include a cyclopropyl group, and a cyclohexyl group.

As the alkenyl group, a linear, branched, or cyclic alkenyl group having from 2 to 10 carbon atoms can be exemplifid. Specific examples of the alkenyl group include a vinyl group, a propenyl group, a butenyl group, a pentenyl group, a hexenyl group, a heptenyl group, an octenyl group, a nonenyl group, and an allyl group.

As the aryl group, an aryl group having from 6 to 12 carbon atoms can be exemplifid. Specific examples of the aryl group include phenyl, 1-naphthyl, 2-naphthyl, and biphenyl.

As the heteroaryl group, a heteroaryl group having from 3 to 10 carbon atoms can be exemplifid. Specific examples of the heteroaryl group include 1,2,3-triazole, imidazole, and thiophene.

As the alkynyl group, a linear, or branched alkynyl group having from 2 to 10 carbon atoms can be exemplifid. Specific examples of the alkynyl group include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

As the acyl group, for example, a linear or branched alkanoyl having from 1 to 10 carbon atoms can be exemplifid. Specific examples of the acyl group include an acetyl group, a n-propionyl group, an isopropionyl group, a n-butyryl group, and a hexanoyl group.

Among the substituents described above, from the viewpoint of the double-strand forming ability, an alkyl group having from 2 to 5 carbon atoms, an alkenyl group having from 2 to 5 carbon atoms, an alkynyl group having from 2 to 5 carbon atoms, and a halogenyl group are preferred, further, an alkynyl group having from 2 to 5 carbon atoms, and a halogenyl group are more preferred, and an alkynyl group having from 3 to 4 carbon atoms is particularly preferred.

In the General Formula (2), each of R² and R³ may be independently a hydrogen atom, or an alkyl group having from 1 to 10 carbon atoms. As the alkyl group, for example, a linear or branched alkyl group having from 1 to 10 carbon atoms can be exemplifid. Specific examples of the alkyl group include a methyl group, an ethyl group, a n-propyl group, an i-propyl group, a n-butyl group, a t-butyl group, a n-hexyl group, and a n-octyl group.

Further, R² and R³ may be bonded to each other to form a ring. Specifically, for example, R²R³, and a carbon atom other than R³ to which R² is bonded may form a cyclic structure such as a cyclopropane ring, a cyclobutane ring, a cyclohexane ring, or the like.

In addition, from the viewpoint of the double-strand forming ability, the pyrimidine base in the General Formulae (1) and (2) is a group represented by the following General Formula (3) or (4).

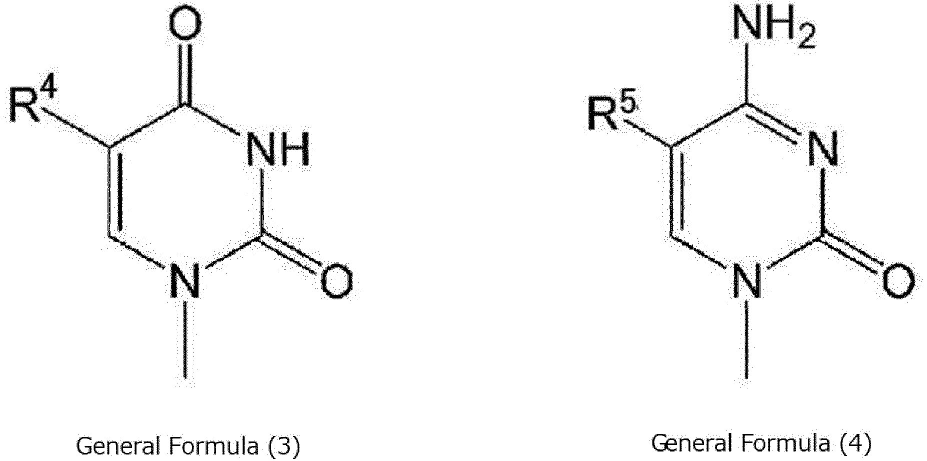

According to the present invention, in General Formula (3), R⁴ represents a propynyl group, and each of a carbonyl group and an amino group in General Formula (3) may have a protecting group; and in General Formula (4), R⁵ represents a propynyl group, and each of a carbonyl group and an amino group in General Formula (4) may have a protecting group. Described and not claimed *per se* is that in General Formulae (3) and (4), each of R⁴ and R⁵ independently represents a hydrogen atom, an alkyl group having from 1 to 10 carbon atoms, an alkenyl group having from 2 to 10 carbon atoms, an alkynyl group having from 2 to 10 carbon atoms, or a halogenyl group. Each of a carbonyl group and an amino group in General Formulae (3) and (4) may have a protecting group.

As the alkyl group having from 1 to 10 carbon atoms, a linear or branched alkyl group having from 1 to 10 carbon atoms can be exemplifid. Specific examples of the alkyl group having from 1 to 10 carbon atoms group include a methyl group, an ethyl group, a n-propyl group, an i-propyl group, a n-butyl group, and t-butyl group.

Examples of the alkenyl group having from 2 to 10 carbon atoms include a vinyl group, a propenyl group, a butenyl group, a pentenyl group, and a hexenyl group.

Examples of the alkynyl group having from 2 to 10 carbon atoms include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, and a 1-methyl-2-butynyl group.

Among them, each of R⁴ and R⁵ is preferably an alkyl group having from 2 to 5 carbon atoms, an alkenyl group having from 2 to 5 carbon atoms, or an alkynyl group having from 2 to 5 carbon atoms, and is particularly preferably an alkyl group having from 3 to 4 carbon atoms, an alkenyl group having from 3 to 4 carbon atoms, or an alkynyl group having from 3 to 4 carbon atoms.

In addition, the protecting group is not particularly limited,any known protecting group can be used, and examples of the protecting group in the General Formula (4) include an acetyl group, an isobutyl group, a benzoyl group, and a dimethylaminomethylene group. The protecting group is appropriately adjusted depending on the kind, the length, or the like of the base sequence of RNA to be targeted.

Further, from the viewpoint of the double-strand forming ability with RNA, each Bs₁ in General Formulae (1) and (2) is a base represented by General Formula (3) among the General Formulae (3) and (4).

The production method of the nucleic acid oligomer for RNA hybrid formation described above is not particularly limited as long as the nucleic acid oligomer for RNA hybrid formation can be produced so as to contain from 3 to 50 specific nucleotide continuous units. However, from the viewpoint of the ease of production, it is preferred that the nucleic acid oligomer for RNA hybrid formation is produced by performing a process of synthesizing a monomer capable of constituting a specific nucleotide unit (monomer synthesis process), and then by performing a process (condensation process) of sequentially condensing the monomer obtained by the monomer synthesis process.

In the monomer synthesis process, referring to, for example, the synthesis method of a monomer described in a document of [J. Am. Chem. Soc., Vol. 130, pp. 16031-16037] or in JP-A No. 2015-44842 as the synthesis method of a monomer for forming a nucleotide unit represented by the General Formula (1), a monomer capable of being used in the subsequent condensation process can be obtained by synthesizing a derivative of a nucleotide having an appropriate substituent introduced at the 5-position of a pyrimidine base, in which absolute steric configuration of a phosphorus atom is controlled. In addition, referring to a known method of synthesizing a nucleobase unit of Locked Nucleic acid (LNA), for example, a synthesis method of a monomer described in JP-A No. 2015-093853, or the like as the synthesis method of a monomer for forming a nucleotide unit represented by the General Formula (2), a monomer can be synthesized.

In this regard, it is preferred that a monomer having a stereochemical purity of, for example, 97% or more as the stereochemical purity of a monomer to be used in the following condensation process is used.

In the condensation process, for example, referring to a document of Wada, et al., [J. Org. Chem. Vol. 77, 7913 (2012)], a method of sequentially condensing a monomer obtained in the monomer synthesis process can be used so that a desired base sequence and a desired kind of nucleotide units can be obtained. That is, in consideration of a base sequence of RNA to be targeted, a nucleic acid oligomer for RNA hybrid formation can be obtained by condensing the monomer.

In the condensation reaction of a monomer described in the document, condensation can be performed while maintaining the absolute steric configuration of a phosphorus atom high, and therefore, by conducting the condensation reaction by using a monomer obtained in the monomer synthesis process, the nucleic acid oligomer for RNA hybrid formation according to the present invention, which has a high stereochemical purity, can be obtained as a result. In addition, in the condensation of monomer, as for the kind of a substituent at the 5-position of a pyrimidine base in a monomer to be used, for example, a monomer having a base to which substituents all being constant are bonded may be used, or a monomer having a base to which substituents different from each other are bonded may be used.

In a case in which the nucleic acid oligomer for RNA hybrid formation is a chimeric oligomer, a monomer is synthesized by the monomer synthesis process described above, and then in the condensation process, in consideration of a base sequence of RNA to be targeted, the nucleic acid oligomer for RNA hybrid formation can be synthesized by appropriate condensation using, for example, both of the monomer and a monomer capable of constituting another nucleotide unit. Specifically, referring to a method described in, for example, a document of Nukaga, et al., [Yohei Nukaga; Natsuhisa Oka; Yusuke Maeda; and Takeshi Wada, "Solid Phase Synthesis of PS/PO Chimeric Oligonucleotides Stereocontrolled with Respect of Phosphorus Atom", antisense, gene, delivery symposium 2014, Lecture Proceedings, p. 52], or a document of Oka, et al., [J. Am. Chem. Soc., Vol. 130, pp. 16031-16037], the nucleic acid oligomer for RNA hybrid formation can be obtained by appropriately condensing the monomer and a monomer capable of constituting another nucleotide unit to the monomer obtained by the monomer synthesis process.

In the method for producing the nucleic acid oligomer for RNA hybrid formation, after a condensation process, a protecting group elimination process may be appropriately provided. Examples of the deprotecting agent capable of being used in a protecting group elimination process include trifluoroacetic acid, trichloroacetic acid, and dichloroacetic acid.

The nucleic acid oligomer obtained in the method described above can be purified, for example, by a known purification method of reversed-phase high performance liquid chromatography (reversed-phase HPLC), ion exchange HPLC, column chromatography, recrystallization, or the like.

The nucleic acid oligomer for RNA hybrid formation according to the present invention can be used as an antisense molecule that is excellent in the double-strand forming ability to the RNA to be targeted by being designed to be complementary to the base sequence of RNA to be targeted. For example, in a case in which RNA to be targeted corresponds to a partial sequence of a disease-related gene, the nucleic acid oligomer for RNA hybrid formation according to the present invention is preferably used for medicinal use of an antisense medicine having high translation inhibiting ability, or the like.

### Examples

Hereinafter, the present invention is specifically described by way of Examples, however, the present invention is not limited to these Examples.

Various analyzers shown below were used.
¹H-nuclear magnetic resonance spectrum (¹H-NMR): JNM-LA 400 (400 MHz)
¹³C-nuclear magnetic resonance spectrum (¹³C-NMR): JNM-LA 400 (100.5 MHz)
³¹P-nuclear magnetic resonance spectrum (³¹P-NMR): JNM-LA 400 (161.8 MHz)

In addition, as an internal standard, tetramethylsilane (TMS) was used for ¹H-NMR, and CDCl₃ (δ 77.16 ppm) was used for ¹³C-NMR, and 85% H₃PO₄ was used for ³¹P-NMR as an external standard.

### Electrospray ionization mass spectrometry (ESI MS): Varian 910-MS

### Ultraviolet and visible spectrophotometer: JASCO V-550 UV/VIS spectrophotometer

Note that the expression "%" is on a mass basis unless otherwise specifically noted. In Examples, HNEt₃ represents triethylamine, DMTr represents 4,4'-dimethoxytrityl, and other abbreviations are synonymous with those in the above description.

### <Synthesis of Oxazaphospholidine Monomer>

An oxazaphospholidine monomer was synthesized by reacting each of the nucleosides protected with a DMTr group (compounds 3a to 3e) with a compound derived from an amino alcohol (compound D2 or compound L2).

Hereinafter, the present invention will be described in detail.

### (Synthesis of 2-chlorooxazaphospholidine D2)

A compound D1 (1.77 g, 10 mmol) being an amino alcohol was repeatedly azeotropically dried with toluene, and 2.20 mL (20 mmol) of methylmorpholine was added into 5 mL of the toluene solution to obtain a mixture solution. Into the mixture solution, a 5.0 mL solution of toluene containing 870 µL (10 mmol) of phosphorus trichloride was added while being stirred at 0°C, and further reaction of the resultant mixture was conducted while being stirred at room temperature for 2 hours. The salt generated after the reaction was separated by filtration at -78°C under an Ar atmosphere, and concentrated under reduced pressure in an argon atmosphere to obtain 2.09 g of yellow oily 2-chloro-1,3,2-oxazaphospholidine (Compound D2). The obtained compound D2 was not further purified, and was used for the synthesis of the subsequent compounds.

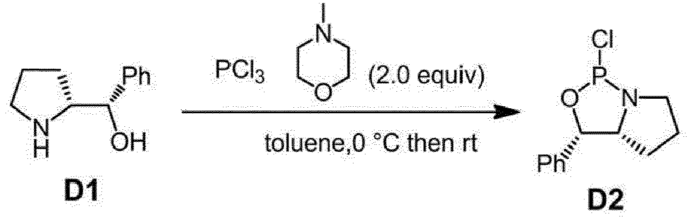

Further, L2 being a diastereomer of D2 was synthesized in a similar manner as that described above by using a compound L1 in place of the compound D1.

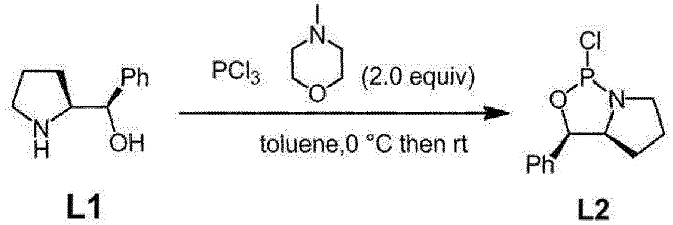

### (Preparation of Compounds from 3a to 3e)

Hereinafter, by referring to a method described in a document [J. Am. Chem. Soc., Vol. 104, 1316-1319], 5'-O-di(p-methoxylphenyl)phenylmethyl(DMTr)-2'-deoxyuridine (compound 3a), 5'-O-di(p-methoxylphenyl)phenylmethyl(DMTr)-thymidine (compound 3b), 5'-O-di(p-methoxylphenyl)phenylmethyl(DMTr)-2'-deoxybromouridine (compound 3c), 5'-O-di(p-methoxylphenyl)phenylmethyl(DMTr)-2'-deoxyiodouridine (compound 3d), and 5'-O-di(p-methoxylphenyl)phenylmethyl(DMTr)-2'-deoxypropynyluridine (compound 3e), which are used for synthesizing respective oxazaphospholidine monomers, were synthesized from 2'-deoxyuridine (manufactured by Tokyo Chemical Industry Co., Ltd. (TCI)), thymidine (manufactured by Wako Pure Chemical Industries, Ltd.), 2'-deoxybromouridine (manufactured by Wako Pure Chemical Industries, Ltd.), and 2'-deoxyiodouridine (manufactured by Wako Pure Chemical Industries, Ltd.). By referring to a method described in Tetrahedron Lett., Vol. 45, 2457-2461, 5'-O-di(p-methoxylphenyl)phenylmethyl(DMTr)-2'-deoxypropynyluridine (compound 3e) was synthesized from 5'-O-di(p-methoxylphenyl)phenylmethyl(DMTr)-2'-deoxyiodouridine (compound 3d).

### (Synthesis of Oxazaphospholidine Monomer)

### <Synthesis of Compound (Sp)-4a>

Compound 3a (531 mg, 1.0 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which a compound D2 (725 mg) had been dissolved was added dropwise into the cooled mixture, and then the resultant mixture was stirred at room temperature for 2 hours. After that, into the mixture, 300 mL of chloroform, and a saturated hydrogen carbonate aqueous solution (100 mL) were added, the resultant mixture was subjected to liquid separation operation, the organic phase was washed twice with 100 mL of a saturated hydrogen carbonate aqueous solution, further, chloroform was added into the recovered washing solution, then the resultant mixture was subjected to liquid separation operation, and the organic layer was collected. All of the collected organic layers were dried over anhydrous sodium sulfate, then filtered, and the solvent was distilled off under reduced pressure to obtain a residue. The obtained residue was separated and purified by silica gel column chromatography (16 g of NH-silica gel, manufactured by FUJI SILYSIA CHEMICAL LTD.) [eluent: toluene/ethyl acetate/triethylamine (70/30/0.1, v/v/v)], and then the solvent was distilled off under reduced pressure to obtained (Sp)-4a (313 mg, 0.43 mmol) in a yield of 43%. 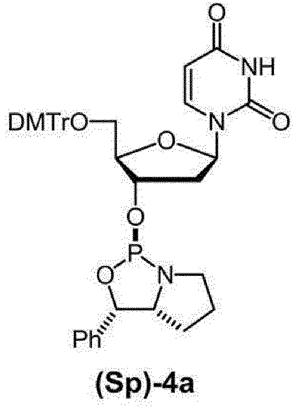 ¹H NMR(400 Hz, CDCl₃), δ7.88-7.86(d, 1H), 7.41-7.18(m, 37H), 6.85-6.83(d, 7H), 6.32-3.29(t, 1H), 5.74-5.73(d, 1H), 5.34-5.31(d, 1H), 4.94(m, 1H), 4.16(m, 1H), 3.89(m, 1H), 3.79(s, 6H), 3.59(m, 2H), 3.18(m, 1H), 2.76(br, 1H), 2.49(m, 1H), 2.34(m, 1H), 1.85(m, 2H), 1.65(m, 1H), 1.25-1.16(m, 4H), 0.99(m, 1H)
³¹P NMR(400 Hz, CDCl₃), δ156.89

### <Synthesis of Compound (Sp)-4b>

Compound 3b (544 mg, 1.0 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound D2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (70/30/0.1, v/v/v)], and then the solvent was distilled off under reduced pressure to obtained (Sp)-4b (330 mg, 0.44 mmol) in a yield of 44%.

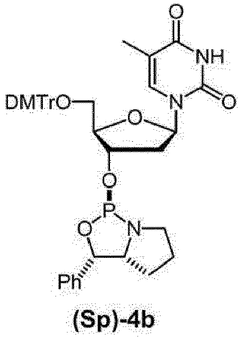

### <Synthesis of Compound (Sp)-4c>

Compound 3c (611 mg, 1.0 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound D2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (from 80/20/0.5 (v/v/v) to 0/100/0.5 (v/v/v))], and then the solvent was distilled off under reduced pressure to obtain Compound (Sp)-4c (280 mg, 0.44 mmol) in a yield of 30%. 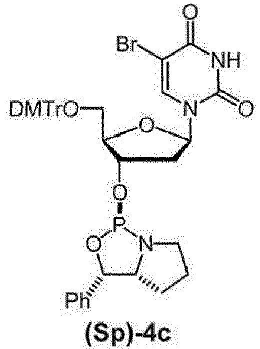 ¹H NMR(400 Hz, CDCl₃), δ81.2(s, 1H), δ7.44-7.23(d, 19H), 6.82-6.84(d, 4H), 6.32(t, 1H), 5.71-5.70(d, 1H), 4.91(m, 1H), 4.21(m, 1H), 3.89 (m, 1H), 3.78(s, 6H), 3.55 (m, 1H), 3.(m, 1H), 2.53(m, 1H), 2.33(m, 1H), 1.64(m, 2H), 1.28- 1.20(m, 1H), 1.01-0.96(m, 1H)
³¹P NMR(400 Hz, CDCl₃), δ156.10

### <Synthesis of Compound (Sp)-4d>

Compound 3d (658 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound D2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (17 g of NH-silica gel, manufactured by FUJI SILYSIA CHEMICAL LTD.) [eluent: toluene/ethyl acetate/triethylamine (from 80/20/0.1 (v/v/v) to 0/100/0.1 (v/v/v))], and then the solvent was distilled off under reduced pressure to obtain Compound (Sp)-4d (420 mg,0.49 mmol) in a yield of 49%. 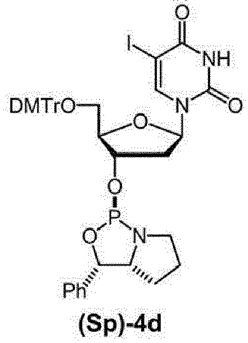 ¹H NMR(400 Hz, CDCl₃), δ8.17(s, 1H), δ7.46-7.25(d, 19H), 6.87-6.85(d, 4H), 6.32(t, 1H), 5.70-5.68(d, 1H), 4.89(m, 1H), 4.21(m, 1H), 3.92-3.89(m, 1H), 3.79(s, 6H), 3.55(m, 1H), 3.48(dd, 1H), 3.37(dd, 1H), 2.89(m, 1H), 2.56-2.50(m, 1H), 2.36-2.23(m, 1H), 1.67-1.56(m, 2H), 1.03-0.95(m, 1H)
³¹P NMR(400 Hz, CDCl₃), δ155.55

### <Synthesis of Compound (Sp)-4e>

Compound 3e (612 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound D2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (from 80/20/0.1 (v/v/v) to 0/100/0.1 (v/v/v))], and then the solvent was distilled off under reduced pressure to obtain Compound (Sp)-4e (350 mg, 0.49 mmol) in a yield of 49%. 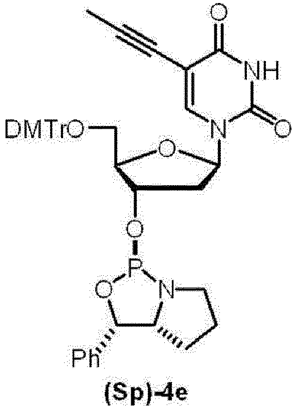 ¹H NMR(400 Hz, CDCl₃), δ8.04(s, 1H), δ7.47-7.23(d, 19H), 6.86-6.84(d, 4H), 6.30(t, 1H), 5.72-5.70(d, 1H), 4.91(m, 1H), 4.21(m, 1H), 3.92-3.89(m, 1H), 3.79(s, 6H), 3.56(m, 1H), 3.42(m, 2H), 3.18(m, 1H), 2.52(m, 1H), 2.33(m, 1H), 1.69-1.52(m, 5H), 1.26-1.20(m, 1H), 1.01-0.96(m, 1H)
³¹P NMR(400 Hz, CDCl₃), δ156.01

### <Synthesis of Compound (Rp)-4a>

Compound 3a (530 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound L2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (70/30/0.1, v/v/v)], and then the solvent was distilled off under reduced pressure to obtain Compound (Rp)-4a (360 mg, 0.49 mmol) in a yield of 49%. 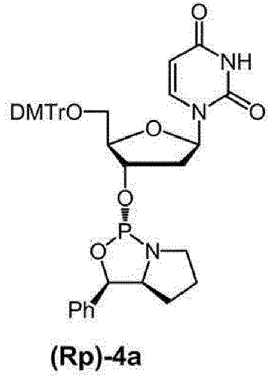 ¹H NMR(400 Hz, CDCl₃), δ7.82-7.81(d, 1H), 7.38-7.23(m, 16H), 6.82-6.79(dd, 4H), 6.33-(t, 1H), 5.75-5.73(d, 1H), 5.31-5.29(d, 1H), 4.95(m, 1H), 4.11(m, 1H), 3.88(m, 1H), 3.77(s, 3H), 3.76(s, 3H), δ3.56(m, 1H), 3.48(dd, H), 3.44(dd, 1H), 3.19(m, 1H), 2.62(m, 1H), 2.34(m, 1H), 1.66(m, 4H), 1.32(m, 1H), 1.19(m, 1H), 0.94(m, 1H)
³¹P NMR(400 Hz, CDCl₃), δ156.87

### <Synthesis of Compound (Rp)-4b>

Compound 3b (545 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound L2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (70/30/0.1, v/v/v)], and then the solvent was distilled off under reduced pressure to obtain Compound (Rp)-4b (350 mg, 0.35 mmol) in a yield of 47%.

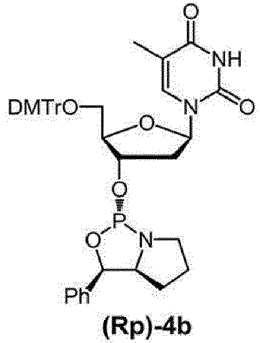

### <Synthesis of Compound (Rp)-4c>

Compound 3c (611 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound L2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (from 80/20/0.1 (v/v/v) to 0/100/0.1 (v/v/v))], and then the solvent was distilled off under reduced pressure to obtain Compound (Rp)-4c (330 mg, 0.41 mmol) in a yield of 41%. 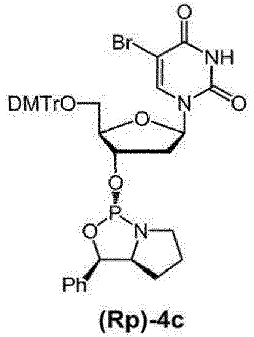 ¹H NMR(400 Hz, CDCl₃), δ8.06(s, 1H), δ7.42-7.21(d, 19H), 6.83-6.80(d, 4H), 6.35(t, 1H), 5.74-5.72(d, 1H), 4.90(m, 1H), 4.15(m, 1H), 3.87-3.83(m, 1H), 3.76(s, 6H), 3.58(m, 1H), 3.39(m, 2H), 3.18(m, 1H), 2.65(m, 1H), 2.33(m, 1H), 1.64(m, 2H), 1.21-1.15(m, 1H), 0.99-0.91(m, 1H)
³¹P NMR(400 Hz, CDCl₃), δ156.46

### <Synthesis of Compound (Rp)-4d>

Compound 3d (657 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound L2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (15 g of NH-silica gel, manufactured by FUJI SILYSIA CHEMICAL LTD.) [eluent: toluene/ethyl acetate/triethylamine (from 80/20/0.1 (v/v/v) to 0/100/0.5 (v/v/v))], and then the solvent was distilled off under reduced pressure to obtain Compound (Rp)-4d (360 mg, 0.42 mmol) in a yield of 42%. 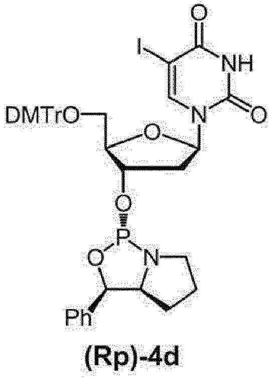 ¹H NMR(400 Hz, CDCl₃), δ8.13 (s, 1H), δ7.43-7.16(d, 22H), 6.83-6.81(d, 4H), 6.34(t, 1H), 5.73-5.71(d, 1H), 4.89(m, 1H), 4.16(m, 1H), 3.85-3.05(m, 1H), 3.77(s, 6H), 3.61-3.55(m, 1H), 3.38(m, 2H), 3.20-3.14(m, 1H), 2.64(m, 1H), 2.32(m, 2H),1.64(m, 2H), 1.21(m, 3H), 1.19-1.15(m, 1H), 0.96-0.91(m, 1H)
³¹P NMR(400 Hz, CDCl₃), δ156.23

### <Synthesis of Compound (Rp)-4e>

Compound 3e (531 mg, 1 mmol) was repeatedly azeotroped with pyridine and toluene to obtain a 5 mL solution of tetrahydrofuran, 0.97 mL (7 mmol) of triethylamine was added and mixed into the 5 mL solution of tetrahydrofuran, the resultant mixture was cooled to -78°C, 6 mL of tetrahydrofuran (THF) in which Compound L2 (725 mg) had been dissolved was added dropwise into the cooled mixture, and then the resultant mixture was stirred at room temperature for 2 hours. After that, a residue was obtained in a similar manner as in the method for Compound (Sp)-4a. The obtained residue was separated and purified by silica gel column chromatography (17 g of NH-silica gel, manufactured by FUJI SILYSIA CHEMICAL LTD.) [eluent: toluene/ethyl acetate/triethylamine (from 80/20/0.1 (v/v/v) to 0/100/0.1 (v/v/v))], and then the solvent was distilled off under reduced pressure to obtain Compound (Rp)-4e (420 mg, 0.29 mmol) in a yield of 40%. 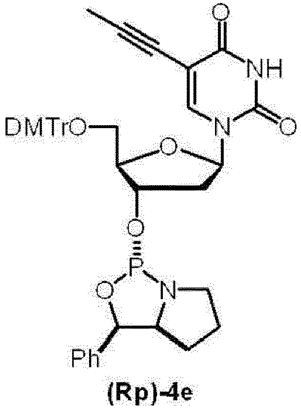 ¹H NMR(400 Hz, CDCl₃), δ7.98(s, 1H), δ7.45-7.20(d, 19H), 6.82-6.80(d, 4H), 6.34(t, 1H), 5.74-5.72(d, 1H), 4.90(m, 1H), 4.15(m, 1H), 3.88-3.84(m, 1H), 3.77(s, 6H), 3.58(m, 1H), 3.38(m, 2H), 3.19(m, 1H), 2.65(m, 1H), 2.33(m, 1H), 1.71-1.61(m, 5H), 1.26-1.16(m, 1H), 0.99-0.92(m, 1H)
³¹P NMR(400 Hz, CDCl₃), δ156.6

### (Preparation of Compounds 5a, 5b and 5e)

Hereinafter, by referring to a method described in a document [J. Am. Chem. Soc., Vol. 104, 1316-1319], 5'-O-di(p-methoxylphenyl)phenylmethyl(DMTr)-2'-deoxycytidine (compound 5a), 5'-O-di(p-methoxylphenyl)phenylmethyl(DMTr)-methylcytidine (compound 5b), 5'-O-di(p-methoxylphenyl)phenylmethyl(DMTr)-2'-deoxyiodocytidine (compound 5d), and 5'-O-di(p-methoxylphenyl)phenylmethyl(DMTr)-2'-deoxypropynylcytidine (compound 5e), which are used for synthesizing respective oxazaphospholidine monomers, were synthesized from 2'-deoxycytidine (manufactured by Tokyo Chemical Industry Co., Ltd. (TCI)), 5'-methylcytidine (synthesized from thymidine by a method described in US 4,754,026), 2'-deoxybromocytidine (manufactured by Wako Pure Chemical Industries, Ltd.), and 2'-deoxyiodocytidine (manufactured by Wako Pure Chemical Industries, Ltd.). By referring to a method described in Tetrahedron Lett., Vol. 45, 2457-2461, 5'-O-di(p-methoxylphenyl)phenylmethyl(DMTr)-2'-deoxypropynylcytidine (compound 5e) was synthesized from 5'-O-di(p-methoxylphenyl)phenylmethyl(DMTr)-2'-deoxyiodocytidine (compound 5d).

### <Synthesis of Compound (Sp)-6a>

Compound (Sp)-6a was synthesized in a similar manner as in the synthesis of Compound (Sp)-4a except that a compound 5a (0.96 g, 1.5 mmol) was used in place of compound 3a. Further, colorless compound (Sp)-6a (0.70 g, 0.84 mmol, yield 56%) was obtained in a similar manner as in the purification of Compound (Sp)-4a except that the conditions of silica gel column chromatography in the purification were changed to[eluent: toluene/ethyl acetate/triethylamine (80/20/0.1, v/v/v)].

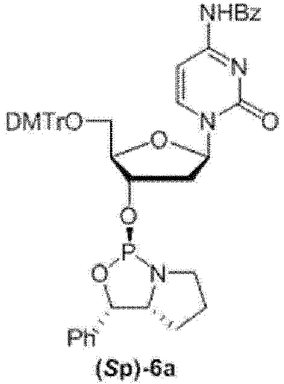

### <Synthesis of Compound (Sp)-6b>

Compound (Sp)-6b was synthesized and purified in a similar manner as in the synthesis of Compound (Sp)-4b except that Compound 5b (0.97 g, 1.5 mmol) was used in place of Compound 3b. Colorless (Sp)-6b (0.74 g, 0.87 mmol, yield 58%) was obtained. 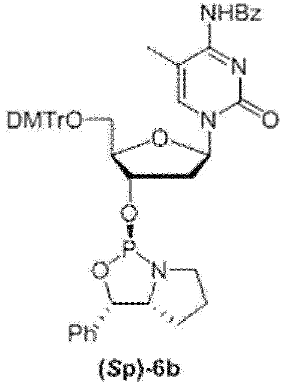 ¹H NMR (300 MHz, CHCl₃) δ 8.29 (d, J = 7.3 Hz, 2H), 7.87 (s, 1H), 7.51-7.26 (m, 17H), 6.85 (d, J = 8.4 Hz, 4H), 6.42 (t, J = 6.4 Hz, 1H), 5.70 (d, J = 6.2 Hz, 1H), 4.98-4.90 (m, 1H), 4.22-4.21 (m, 1H), 3.93-3.85 (m, 1H), 3.79 (s, 6H), 3.60-3.37 (m, 3H), 3.22-3.08 (m, 1H), 2.56-2.45 (m, 1H), 2.44-2.35 (m, 1H), 1.76-1.56 (m, 5H), 1.29-1.16 (m, 1H), 1.04-0.95 (m, 1H)
³¹P NMR (161 MHz, CDCl₃) δ 156.18. FAB-HR MS: Calcd. for [M+H]⁺; 853.3361. Found; 853.3364.

### <Synthesis of Compound (Sp)-6e>

Compound (Sp)-6e was synthesized in a similar manner as in the synthesis of Compound (Sp)-4e except that Compound 5e (0.67 g, 1 mmol) was used in place of Compound 3e, and the amount of compound D2 was changed to 483 mg. Further, the purification was performed in a similar manner as in the purification of a compound (Sp)-4e except that the conditions of silica gel column chromatography in the purification were changed to [eluent: toluene/ethyl acetate/triethylamine (from 90/10/0.5, v/v/v to 80/20/0.5, v/v/v)], and colorless compound (Sp)-6e (0.40 g, 0.45 mmol, yield 45%) was obtained. 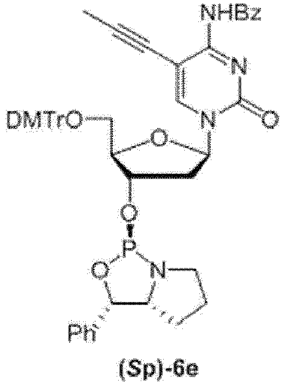 ¹H NMR (400 MHz, CHCl₃) δ 8.33-8.23 (br, 3H), 7.53-7.23 (m, 17H), 6.85 (d, J = 8.8 Hz, 4H), 6.34-6.29 (m, 1H), 5.72 (d, J= 6.3 Hz, 1H), 4.95-4.89 (m, 1H), 4.24 (m, 1H), 3.94-3.87 (m, 1H), 3.78 (s, 6H), 3.61-3.51 (m, 1H), 3.49-3.38 (m, 2H), 3.23-3.14 (m, 1H), 2.65-2.59 (m, 1H), 2.42-2.35 (m, 1H), 1.72- 1.59 (m, 5H), 1.26 -1.18 (m, 1H), 1.03-0.94 (m, 1H)
³¹P NMR (161 MHz, CDCl₃) δ 156.50. FAB-HR MS: Calcd. for [M+H]⁺; 877.3361. Found; 877.3364.

### <Synthesis of Compound (Rp)-6a>

Compound (Rp)-6a was synthesized in a similar manner as in the synthesis of Compound (Rp)-4a except that Compound 5a (1.27 g, 2 mmol) was used in place of Compound 3a, and the amount of Compound L2 was changed to 967 mg. Further, the purification was performed in a similar manner as in the purification of Compound (Rp)-4a except that the conditions of silica gel column chromatography in the purification were changed to [eluent: toluene/ethyl acetate/triethylamine (from 90/10/0.1, v/v/v to 80/20/0.1, v/v/v)], and colorless (Rp)-6a was obtained (0.54 g, 0.65 mmol, yield 33%).

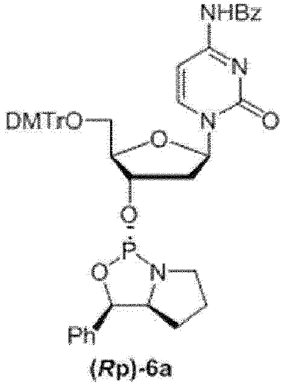

### <Synthesis of Compound (Rp)-6b>

Compound (Rp)-6b was synthesized in a similar manner as in the synthesis of Compound (Rp)-4b except that Compound 5b (0.97 g, 1.5 mmol) was used in place of Compound 3b. Further, the purification was performed in a similar manner as in the purification of Compound (Rp)-4b except that the conditions of silica gel column chromatography in the purification were changed to [eluent: toluene/ethyl acetate/triethylamine (from 90/10/0.1, v/v/v to 80/20/0.1, v/v/v)], and colorless (Rp)-6b was obtained (0.54 g, 0.63 mmol, 42%). 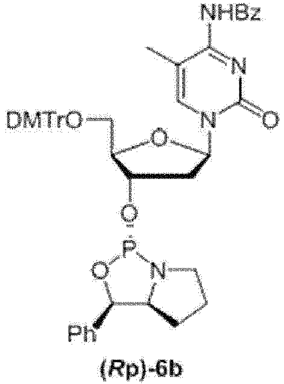 ¹H NMR (400 MHz, CHCl₃) δ 8.29 (d, J = 7.3 Hz, 2H), 7.81 (s, 1H), 7.50-7.23 (m, 17H), 6.81 (dd, J = 8.9, 2.1 Hz, 4H), 6.44 (t, J = 6.6 Hz, 1H), 5.72 (d, J = 6.3 Hz, 1H), 4.96-4.90 (m, 1H), 4.17-4.15 (m, 1H), 3.86-3.79 (m, 1H), 3.78 (s, 6H), 3.62-3.54 (m, 1H), 3.52-3.39 (m, 2H), 3.14-3.22 (m, 1H), 2.68 -2.62 (m, 1H), 2.43-2.35 (m, 1H), 1.67-1.57 (m, 5H), 1.19 -1.15 (m, 1H), 0.98-0.90 (m, 1H)
³¹P NMR (161 MHz, CDCl₃) δ156.55. FAB-HR MS: Calcd. for [M+H]⁺; 853.3361. Found; 853.3361.

### <Synthesis of Compound (Rp)-6e>

Compound (Rp)-6e was synthesized in a similar manner as in the synthesis of Compound (Rp)-4e except that Compound 5e (0.68 g, 1 mmol) was used in place of Compound 3e, and the amount of Compound L2 was changed to 483 mg. Further, the purification was performed in a similar manner as in the purification of Compound (Rp)-4e except that the conditions of silica gel column chromatography in the purification were changed to [eluent: toluene/ethyl acetate/triethylamine (80/20/0.5, v/v/v)], and colorless (Rp)-6e was obtained (0.53 g, 0.60 mmol, yield 60%). 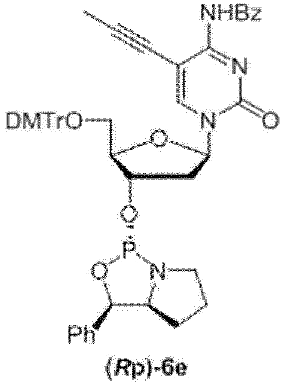 ¹H NMR (400 MHz, CHCl₃) δ 8.25-8.22 (m, 3H), 7.55-7.20 (m, 17H), 6.81 (d, J = 8.8 Hz, 4H), 6.34 (t, J = 6.3 Hz, 1H), 5.73 (d, J = 6.3 Hz, 1H), 4.93-4.87 (m, 1H), 4.19-4.18 (m, 1H), 3.90-3.83 (m, 1H), 3.76 (s, 7H), 3.63-3.53 (m, 1H), 3.40 (d, J = 2.9 Hz, 2H), 3.23-3.14 (m, 1H), 2.77-2.73 (m, 1H), 2.42-2.35 (m, 1H), 1.75 (s, 3H), 1.67-1.61- (m, 2H), 1.22-1.14 (m, 1H), 0.99-0.90 (m, 1H)
³¹P NMR (161 MHz, CDCl₃) δ 156.65. FAB-HR MS: Calcd. for [M+H]⁺; 877.3361. Found; 877.3366.

The yield and purity of the oxazaphospholidine monomer obtained in the above are shown in the following Table 1. In this regard, the stereochemical purity of each of all the compounds obtained above was 99% or more.

**[Table 1]**

| Compound name | Configuration | Substituent | Yield (%) | Purity |
|---|---|---|---|---|
| (Sp)-4a | Sp | H | 43 | 98 |
| (Sp)-4b | Sp | Methyl | 44 | >99 |
| (Sp)-4c | Sp | Bromine | 49 | 99 |
| (Sp)-4d | Sp | Iodine | 49 | 96 |
| (Sp)-4e | Sp | Propynyl | 49 | 98 |
| (Rp)-4a | Rp | H | 49 | 98 |
| (Rp)-4b | Rp | Methyl | 60 | 98 |
| (Rp)-4c | Rp | Bromine | 41 | 97 |
| (Rp)-4d | Rp | Iodine | 42 | 97 |
| (Rp)-4e | Rp | Propynyl | 40 | >99 |
| (Sp)-6a | Sp | H | 56 | >99 |
| (Sp)-6b | Sp | Methyl | 58 | >99 |
| (Sp)-6e | Sp | Propynyl | 45 | >99 |
| (Rp)-6a | Rp | H | 33 | >99 |
| (Rp)-6b | Rp | Methyl | 42 | >99 |
| (Rp)-6e | Rp | Propynyl | 60 | >99 |

From the results described above, all of the monomers were obtained with high purity and high stereochemical purity.

### <Synthesis of Nucleic Acid Oligomer for RNA Hybrid Formation>

In accordance with a method described in a document of Nukaga, et al., [Yohei Nukaga; Natsuhisa Oka; Yusuke Maeda; and Takeshi Wada, "Solid Phase Synthesis of PS/PO Chimeric Oligonucleotides Stereocontrolled with Respect of Phosphorus Atom", antisense, gene, delivery symposium 2014, Lecture Proceedings, p. 52], or a document of Oka, et al., [J. Am. Chem. Soc. 130, pp. 16031-16037], a phosphorothioate type nucleic acid oligomer that is a chimeric oligomer having natural forms of CG base sequences at the 5' end and the 3' end, respectively (5'-CGTTTTTTTTCG-3', 12 mer), a phosphorothioate type nucleic acid oligomer that is a chimeric oligomer having natural forms of ATA and TAT base sequences at the ends, respectively (5'-ATACCCCCCTAT-3', 12 mer), or a nucleic acid oligomer bonded by a phosphate bond in place of the phosphorothioate bond was synthesized by using various kinds of oxazaphospholidine monomers obtained in the above. In this regard, in the case of using a monomer ((SP)-4C, (RP)-4C, (SP)-4d, and (RP)-4d), which is a halogen modification, the synthesis was performed according to the conditions of "ultra mild" described in the above document.

Some of specific conditions are shown in Table 2.

**[Table 2]**

| | | Phosphate type (natural form) nucleic acid oligomer synthesis | | Phosphorothioate type nucleic acid oligomer synthesis | |
|---|---|---|---|---|---|
| Process | Operation | Reagent | Time | Reagent | Time |
| 1 | Detritylation | 3% (w/v) TCA (trichloroacetic acid) in CH₃Cl₂ | 12 s | 3% (w/v) TCA in CH₂Cl₂ | 12 s |
| 2 | Washing | dry CH₃CN | - | dry CH₃CN | - |
| 3 | Condensation | 0.1 M phosphoramidite monomer and 0.25 M ETT (5-ethylthio-1 tetrazole) in dry CH₃CN | 30 s | 0.1 M oxazaphospholidine monomer and 0.25 M ETT in dry CH₃CN | 10 min |
| 4 | Washing | dry CH₃CN | - | dry CH₃CN | - |
| 5 | Capping | Ac₂O or Pac₂O and 16% (v/v) N-methylimidazole (NMI) in dry tetrahydrofuran (THF) | 40 s | 0.5 M N-trifluoroacetylimidazole (CF₃COIm) and 16% (v/v) NMI in dry THF | 40 s |
| 6 | Washing | dry CH₃CN | - | dry CH₃CN | - |
| 7 | Oxidation/Sulfurization | 1 M t-BuOOH in dry toluene | 30 s | 0.3 M 3-phenyl-1,2,4-dithiazoline-one (POS) in dry CH₃CN | 8 min |
| 8 | Washing | dry CH₃CN | - | dry CH₃CN | - |

In addition, all of the nucleic acid oligomers (12 mer) obtained by the synthesis method described above were separated and purified by the following reversed-phase HPLC (RP-HPLC). The RP-HPLC and the separation and purification were performed by using a reverse phase column (SOUCE™ 5RPC ST 5 µm column (5 µm, 4.6 mm × 150 mm)) connected to a HPLC device (Box-900) manufactured by GE Healthcare under the conditions of a room temperature, a flow rate of 1.0 mL/min, and using as an eluent a 0.1 M triethylammonium carbonate buffer solution (pH 7.0) with an acetonitrile linear gradient of from 5 to 26%.

Hereinafter, obtained nucleic acid oligomers and measurement results by ESI MS are shown in Tables 3-1 and 3-2. In addition, in the names of the oligomers in Tables 3-1 and 3-2, the expressions "(Sp)" and "(Rp)" each indicate that the absolute steric configuration of a phosphorothioate bond in an oligomer is either one of Sp and Rp.

Further, the expression "ps" indicates that nucleotide residues (T) each having a 5-methyluracil group are bonded to each other by a phosphorothioate bond, nucleotide residues (U) each having a uracil group are bonded to each other by a phosphorothioate bond, nucleotide residues (C) each having a cytidylyl group are bonded to each other by a phosphorothioate bond, nucleotide residues each having a 5-position modified uracil group are bonded to each other by a phosphorothioate bond, or nucleotide residues each having a 5-position modified cytidylyl group are bonded to each other by a phosphorothioate bond, and for example, the expression "(Ups)8" indicates that nucleotide residues each having continuous 8 uracil groups are bonded to each other by a phosphorothioate bond. Furthermore, the expression "(Tps)8" indicates that nucleotide residues each having continuous 8 5-methyluracil groups are bonded to each other by a phosphorothioate bond, the expression "(Cps)6" indicates that nucleotide residues each having continuous 6 cytidylyl groups are bonded to each other by a phosphorothioate bond, the expression "(Cps)2" indicates that nucleotide residues each having continuous 2 cytidylyl groups are bonded to each other by a phosphorothioate bond. The expression "(^{Br}Ups)8" indicates that nucleotide residues each having 8 uracil groups in each of which a bromine atom (Br) is bonded at the 5-position are bonded to each other by a phosphorothioate bond, the expression "(^{I}Ups)8" indicates that nucleotide residues each having 8 uracil groups in each of which an iodine atom (I) is bonded at the 5-position are bonded to each other by a phosphorothioate bond, the expression "(^{p}Ups)8" indicates that nucleotide residues each having 8 uracil groups in each of which a propynyl group is bonded at the 5-position are bonded to each other by a phosphorothioate bond, and the expression "(^{Me}Cps)6" indicates that nucleotide residues each having 6 cytidylyl groups in each of which a methyl group (Me) is bonded at the 5-position are bonded to each other by a phosphorothioate bond. The expression "(Cps)2(^{p}Cps)2(Cps)2" indicates that a nucleotide residue having 2 cytidylyl groups, a nucleotide residue having 2 cytidylyl groups in each of which a propynyl group is bonded at the 5-position, and a nucleotide residue having 2 cytidylyl groups are bonded in this order by a phosphorothioate bond.

Moreover, in all of the oligomers in Tables 3-1 and 3-2, the expression "CG" indicates that 2 residues of cytosine (C) and guanine (G) are bonded in the natural form at the 5'-position and the 3'-position of the oligomer, respectively, and the expression "ATA" or "TAT" indicates that 3 residues of adenine (A) and thymine (T) are bonded in the natural form at the 5'-position and the 3'-position of the oligomer, respectively.

**[Table 3-1]**

| Number | Nucleic acid oligomer | Yield (%) | Measurement results by ESI MS | |
|---|---|---|---|---|
| | | | Theoretical value [M-H]⁻⁵ | Measurement value |
| 1 | (Sp)-d(CG(Ups)8CG | 20 | 723.4532 | 723.45465 |
| 2 | (Rp)-d(CG(Ups)8CG | 28 | 723.4532 | 723.45498 |
| 3 | (Sp)-d(CG(Tps)8CG | 19 | 745.87824 | 745.87925 |
| 4 | (Rp)-d(CG(Tps)8CG | 24 | 745.87824 | 745.88045 |
| 5 | (Sp)-d(CG(^{Br}Ups)8CG | 10 | 850.10887 | 850.1089 |
| 6 | (Rp)-d(CG(^{Br}Ups)8CG | 2 | 850.10887 | 850.10992 |
| 7 | (Sp)-d(CG(^{I}Ups)8CG | 6 | 924.88821 | 924.88783 |
| 8 | (Sp)-d(CG(^{P}Ups)8CG | 5 | 784.27824 | 784.27943 |
| 9 | (Rp)-d(CG(^{P}Ups)8CG | 29 | 784.27824 | 784.27975 |

**[Table 3-2]**

| Number | Nucleic acid oligomer | Yield (%) |
|---|---|---|
| 10 | (Rp)-d(CG(^{I}Ups)8CG | |
| 11 | (Rp)-d(ATA(Cps)8CG | 12 |
| 12 | (Sp)-d(ATA(Cps)8CG | 6 |
| 13 | (Rp)-d(ATA(^{Me}Cps)8CG | 42 |
| 14 | (Sp)-d(ATA(^{Me}Cps)8CG | 11 |
| 15 | (Rp)-d(ATA(^{P}Cps)8CG | 19 |
| 16 | (Sp)-d(ATA(^{P}Cps)6TAT | 12 |
| 17 | (Rp)-d(ATA(Cps)2(^{p}Cps)2(Cps)2TAT) | 42 |
| 18 | (Sp)-d(ATA(Cps)2(^{p}Cps)2(Cps)2TAT) | 14 |

From the measurement results by ESI MS, it was indicated that the nucleic acid oligomers of the above numbers from 1 to 18 (hereinafter, may also be referred to as nucleic acid oligomers from 1 to 18) were obtained. The obtained nucleic acid oligomers from 1 to 18 were applied to the following Examples and Comparative Examples (measurement of a melting temperature of a double-strand with RNA or DNA), and the like.

### (Preparation of mix-d(CG(Tps)8CG))

For mix-d(CG(Tps)8CG), by using a phosphoramidite monomer of 5'-O-di(p-methoxylphenyl)phenylmethyl(DMTr)-2'-deoxythymidine (1 : 1 mixture of Rp isomers and Sp isomers, manufactured by Glen Research Corporation), a phosphorothioate type nucleic acid oligomer was synthesized in a similar manner as that described above. In the mix-d(CG(Tps)8CG, "T" of the (Tps)8 is randomly a Rp isomer or a Sp isomer.

### (Preparation of mix-d(CG(^{p}Ups)8CG))

For mix-d(CG(^{p}Ups)8CG), by using a phosphoramidite monomer of 5-propynyl-5'-O-di(p-methoxylphenyl)phenylmethyl(DMTr)-2'-deoxythymidine (1 : 1 mixture of Rp isomers and Sp isomers, manufactured by Glen Research Corporation), a phosphorothioate type nucleic acid oligomer was synthesized in a similar manner as that described above. In the mix-d(CG(^{p}Ups)8CG), "^{p}U" of the (^{p}Ups)8 is randomly a Rp isomer or a Sp isomer.

### (Preparation of Phosphate Bond Type Nucleic Acid Oligomer)

Phosphate bond type nucleic acid oligomers shown below were prepared.
- d(CG(U)8CG)
- d(CG(T)8CG)
- d(CG(^{Br}U)8CG)
- d(CG(^{I}U)8CG)
- d(CG(^{p}U)8CG)
- d(ATA(C)6TAT)
- d(ATA(^{Me}C)6TAT)

The absence of "ps" on a base indicates that nucleotide residues (T) each having a 5-methyluracil group are bonded to each other by a phosphate bond, nucleotide residues (U) each having a uracil group are bonded to each other by a phosphate bond, nucleotide residues (C) each having a cytidylyl group are bonded to each other by a phosphate bond, nucleotide residues each having a 5-position modified uracil group are bonded to each other by a phosphate bond, or nucleotide residues each having a 5-position modified cytidylyl group are bonded to each other by a phosphate bond, for example, the expression "(U)8" indicates that continuous 8 uracil groups are bonded to each other by a natural form phosphate bond. The expression "(T)8" indicates that continuous 8 5-methyluracil groups are bonded to each other by a phosphate bond. The expression "(C)6" indicates that continuous 6 cytidylyl groups are bonded to each other by a phosphate bond. The expression "(^{Br}U)8" indicates that 8 uracil groups in each of which a bromine atom (Br) is bonded at the 5-position are bonded to each other by a phosphate bond, the expression "(^{I}U)8" indicates that 8 uracil groups in each of which an iodine atom (I) is bonded at the 5-position are bonded to each other by a phosphate bond, the expression "(^{p}U)8" indicates that 8 uracil groups in each of which a propynyl group is bonded at the 5-position are bonded to each other by a phosphate bond, and the expression "(^{Me}C)6" indicates that 6 cytidylyl groups in each of which a methyl group is bonded at the 5-position are bonded to each other by a phosphate bond.

### (Preparation of Target Nucleic Acid)

For the natural forms of RNA and DNA to be target nucleic acids, an RNA oligomer (SEQ ID NO: 1) and a DNA oligomer (SEQ ID NO: 2) of the base sequences shown in SEQ ID NOs: from 1 to 2 were obtained by custom synthesis (HPLC grade) from Japan Bio Services Co., LTD.

### <Evaluation>

### <Examples 1 to 5 and Comparative Examples 1-1 to 5-3: Double-strand of Each Nucleic Acid Oligomer with RNA, and Reference Examples 1 to 8: Measurement of Melting Temperature in Double-strand of Each Nucleic Acid Oligomer with DNA>

### [Examples 1 to 5]

0.4 nmol of each of the Rp isomers of nucleic acid oligomers (nucleic acid oligomers 2, 4, 6, 9, and 10) shown in Examples 1 to 5 in the following Table 3, and 0.4 nmol of a target nucleic acid (natural form complementary strand RNA) (SEQ ID NO: 1) were dissolved in 160 µL of a 10 mM NaH₂PO₄-Na₂HPO₄ and 100 mM NaCl buffer solution (pH 7.0), and 140 µL of the resultant mixture was aliquoted into octuplet cells. After raising the cell temperature of an ultraviolet and visible spectrophotometer from room temperature up to 95°C at a rate of 5°C/min, the state at 95°C was retained for 5 minutes, and the temperature was lowered to 0°C at a rate of -0.5°C/min, and thus annealing was performed. After the oligomer solution was left to stand as it is at 0°C for 30 minutes, a nucleic acid oligomer solution was placed in the cells, the absorbance in 260 nm was measured at 0.25°C intervals under a nitrogen atmosphere while raising the temperature up to 95°C at a rate of 0.5°C/min, and the melting curve was obtained. From the obtained double-strand melting curve, the Tm value was calculated by a median method.

### [Comparative Examples 1-1 to 5-3]

The measurement was performed in a similar manner as in that in Examples 1 to 5 except that the Rp isomers of nucleic acid oligomers (nucleic acid oligomers 2, 4, 6, 9, and 10) were changed to the Sp isomers of nucleic acid oligomers (nucleic acid oligomers 1, 3, 5, 7, and 8) or a phosphate bond type nucleic acid oligomer, and a double-strand melting temperature (Tm) was obtained as shown in Comparative Examples 1-1 to 5-3 in Table 3.

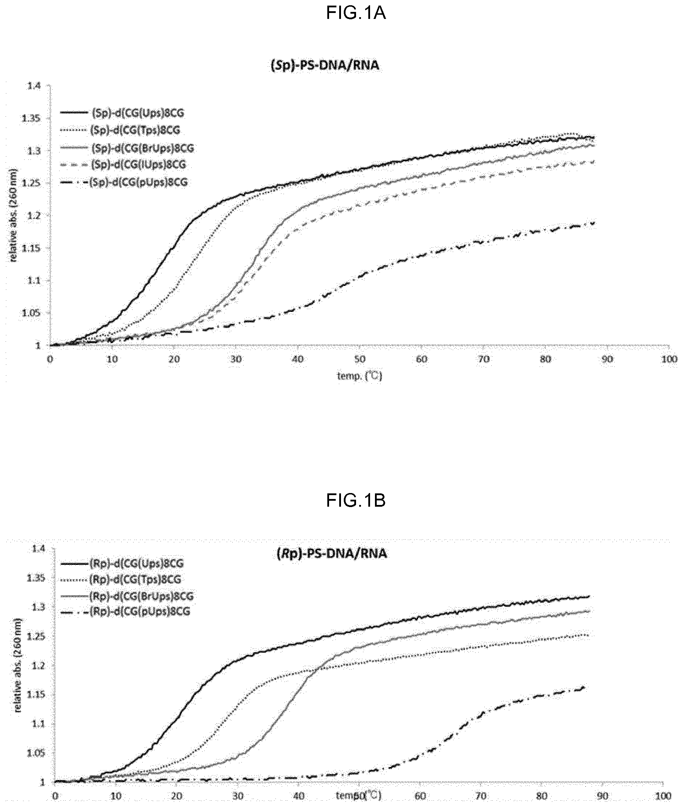

The measurement results are shown in Fig. 1. Fig. 1(A) shows melting curves of double-strands of the Sp isomers of nucleic acid oligomers (nucleic acid oligomers 1, 3, 5, 7, and 8) with a natural form complementary strand RNA, and Fig. 1(B) shows melting curves of double-strands of the Rp isomers of nucleic acid oligomers of (nucleic acid oligomers 2, 4, 6, and 9) with a natural form complementary strand RNA. From these melting curves, the double-strand melting temperature (Tm) was obtained. In this regard, the melting temperature (Tm) was determined by a median method. Specifically, two points were designated in each of the pre-transition and post-transition regions of the obtained melting curve, the baseline was obtained by regression calculation, and the intersection of the median line of two baselines and the melting curve was taken as the melting temperature. The results are shown in Table 3. In addition, the expression "ΔTm (Rp-Sp)" indicates the temperature difference between the Tm of a nucleic acid oligomer being the Rp isomer and the Tm of a nucleic acid oligomer being the Sp isomer. The expression "ΔTm (ps-po)" indicates the temperature difference between the Tm of a phosphorothioate bond oligomer being the Rp or Sp isomer and the Tm of a phosphate bond oligomer. Further, also for random nucleic acid oligomers, 10(mix-PS-dT), mix-d(CG(Tps)8CG, and mix-d(CG(^{p}Ups)8CG, the double-strand melting temperature (Tm) was obtained in a similar manner as in the above.

### [Reference Examples 1 to 8]

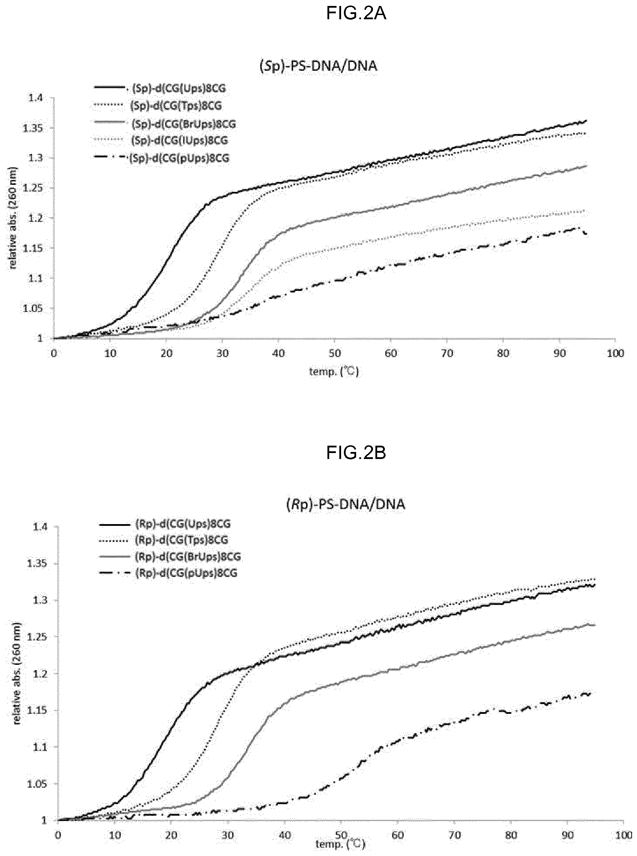

In Reference Examples 1 to 4 and Reference Examples 6 to 8, the measurement was performed in a similar manner as in that in Examples 1 to 5 except that in Examples 1 to 5, the Rp isomers of nucleic acid oligomers (nucleic acid oligomers 2, 4, and 6) was changed to the Sp isomers of nucleic acid oligomers (nucleic acid oligomers 1, 3, 5, and 7), and further the natural form complementary strand RNA being target nucleic acid was changed to a natural form complementary strand DNA (SEQ ID NO: 2), and a double-strand melting temperature (Tm) was obtained as shown in Reference Examples from 1 to 4 and from 6 to 8 in Table 3. In addition, for Reference Example 5, the measurement was performed in a similar manner as in that in Reference Examples 1 to 4 by using the mix-d(CG(Tps)8CG) synthesized in the above, and a double-strand melting temperature (Tm) was obtained as shown in Reference Example 5 in Table 3. Further, the results of the melting curves used for calculating the Tm values of Reference Examples 1 to 4 and 6 to 8 are shown in Fig. 2. Fig. 2(A) shows melting curves of double-strands of the Sp isomers of nucleic acid oligomers (nucleic acid oligomers 1, 3, 5, 7, and 8) with a natural form complementary strand DNA, and Fig. 2(B) shows melting curves of double-strands of the Rp isomers of nucleic acid oligomers of (nucleic acid oligomers 2, 4, 6, and 9) with a natural form complementary strand DNA.

**[Table 4]**

| Kind of target nucleic acid | | Nucleic acid oligomer | Number | Substituent at the 5 - position | Tm°C | ΔTm°C (Rp-Sp) | ΔTm°C (ps-po) |
|---|---|---|---|---|---|---|---|
| Example 1 | RNA | (Rp)-d(CG(Ups)8CF | 2 | H | 20.6 | 2.8 | -12.1 |
| Comparative Example 1-1 | RNA | (Sp)-d(CG(Ups)8CF | 1 | H | 17.8 | | -14.9 |
| Comparative Example 1-2 | RNA | d(CG(Upo)8CG) | | H | 32.7 | | - |
| Example 2 | RNA | (Rp)-d(CG(Tps)8CF | 4 | Methyl | 25.6 | 3.3 | -12.4 |
| Comparative Example 2-1 | RNA | (Sp)-d(CG(Tps)8CF | 3 | Methyl | 22.3 | | -15.7 |
| Comparative Example 2-2 | RNA | d(CG(Tpo)8CG) | | Methyl | 38.0 | | - |
| Comparative Example 2-3 | RNA | mix-d(CG(Tps)8CG | | Methyl | 24.6 | | -13.4 |
| Example 3 | RNA | (Rp)-d(CG(^{Br}Ups)8CF | 6 | Bromine | 37.8 | 5.1 | -10.1 |
| Comparative Example 3-1 | RNA | (Sp)-d(CG(^{Br}Ups)8CF | 5 | Bromine | 32.7 | | -15.2 |
| Comparative Example 3-2 | RNA | d(CG(^{Br}Ups)8CG) | | Bromine | 47.9 | | - |
| Example 4 | RNA | (Rp)-d(CG(^{I}Ups)8CF | 10 | Iodine | 37.9 | 5 . 7 | -11.9 |
| Comparative Example 4-1 | RNA | (Sp)-d(CG(^{I}Ups)8CF | 7 | Iodine | 32.2 | | -17.6 |
| Comparative Example 4-2 | RNA | d(CG(^{I}Ups)8CG) | | Iodine | 49.8 | | - |
| Example 5 | RNA | (Rp)-d(CG(^{P}Ups)8CF | 9 | Propynyl | 61.6 | 16.3 | 1.4 |
| Comparative Example 5-1 | RNA | (Sp)-d(CG(^{P}Ups)8CF | 8 | Propynyl | 45.3 | | -14.9 |
| Comparative Example 5-2 | RNA | d(CG(^{P}Upo)8CG) | | Propynyl | 60.2 | | - |
| Comparative Example 5-3 | RNA | mix-d(CG(^{P}Ups)8CG | | Propynyl | 54.0 | | -6.2 |
| Reference Example 1 | DNA | (Rp)-d(CG(Ups)8CF | 2 | H | 18.0 | -1.8 | |
| Reference Example 2 | DNA | (Sp)-d(CG(Ups)8CF | 1 | H | 19.8 | | |
| Reference Example 3 | DNA | (Rp)-d(CG(Tps)8CF | 4 | Methyl | 27.7 | -1.2 | |
| Reference Example 4 | DNA | (Sp)-d(CG(Tps)8CF | 3 | Methyl | 28.9 | | |
| Reference Example 5 | DNA | mix-d(CG(Tps)8CG | | Methyl | 29.5 | | |
| Reference Example 6 | DNA | (Rp)-d(CG(^{Br}Ups)8CF | 6 | Bromine | 33.0 | -0.4 | |
| Reference Example 7 | DNA | (Sp)-d(CG(^{Br}Ups)8CF | 5 | Bromine | 33.4 | | |
| Reference Example 8 | DNA | (Sp)-d(CG(^{I}Ups)8CF | 7 | Iodine | 34.0 | - | |

As shown in Table 4, in Examples 1 to 5 in which the target nucleic acid was RNA, the Tm values were higher than those in Comparative Examples 1-1 to 5. In particular, it was also indicated that in Example 5 of the Rp isomer, the Tm value was remarkably higher than that in Comparative Example 5-1 of the Sp isomer. Further, it was also indicated that in Example 5, the Tm value was higher than that in Comparative Example 5-2 of a phosphate bond nucleic acid oligomer, and that in a nucleic acid oligomer in which the Rp isomer of a nucleotide and the Sp isomer of a nucleotide were mixed, which is usually used as a phosphorothioate type nucleic acid oligomer. Therefore, it was indicated that a phosphorothioate type nucleic acid oligomer (the Rp isomer) containing a pyrimidine base to which a propynyl group was bonded has higher double-strand forming ability. In addition, in Examples 1 to 4, the Tm value is lower than that of a natural form nucleic acid oligomer, however, the double-strand forming ability is higher than that of a conventional phosphorothioate type nucleic acid oligomer, further, the stability is remarkably higher than that of the natural form nucleic acid oligomer, and therefore, the excellent characteristics as a nucleic acid medicine is provided.

On the other hand, it was indicated that in Reference Examples 1 to 8 in which the target nucleic acid was DNA, the Tm value of the Rp isomer is approximately the same as the Tm value of the Sp isomer, and further, in Reference Examples 1 to 4, 6, and 7, unexpectedly, the Tm value of the Rp isomer is slightly lower than the Tm value of the Sp isomer.

### [Examples 6 to 9]

The measurement results by ESI MS of the following Rp isomers of phosphorothioate type nucleic acid oligomers, which are shown in Table 3, are shown.
- (Rp)-d(ATA(Cps)6TAT) (nucleic acid oligomer 11, Example 6)
   ESI-HR MS: Calcd. for [M]3-; 1205.49151. Found; 1205.49124.
- (Rp)-d(ATA(^{Me}Cps)6TAT) (nucleic acid oligomer 13, Example 7)
   ESI-HR MS: Calcd. for [M]3-; 1233.52281. Found; 1233.52511.
- (Rp)-d(ATA(^{p}Cps)6TAT) (nucleic acid oligomer 15, Example 8)
   ESI-HR MS: Calcd. for [M]4-; 960.89029. Found; 960.89180.
- (Rp)-d(ATA(Cps)2(^{p}Cps)2(Cps)2TAT) (nucleic acid oligomer 17, Example 9)
   ESI-HR MS: Calcd. for [M]3-; 1230.83527. Found; 1230.83978.

The measurement was performed in a similar manner as in that in Examples 1 to 5 except that the nucleic acid oligomers used in Examples 1 to 5 (nucleic acid oligomers 2, 4, 6, 9, and 10) were changed to a nucleic acid oligomer in Example 6, 7, or 9 (nucleic acid oligomer 11, 13, or 17), and that in measuring the Tm value, the temperature was raised up to 95°C at a rate of 0.2°C/min instead of being raised up to 95°C at a rate of 0.5°C/min, and the double-strand melting temperature (Tm) was obtained.

### [Comparative Examples 6-1 to 9-1]

The measurement results by ESI MS of the Sp isomers of phosphorothioate type nucleic acid oligomers shown below, which are described above, are shown.
- (Sp)-d(ATA(Cps)6TAT) (nucleic acid oligomer 12, Comparative Example 6-1)
   ESI-HR MS: Calcd. for [M]3-; 1205.49151. Found; 1205.49371.
- (Sp)-d(ATA(^{Me}Cps)6TAT) (nucleic acid oligomer 14, Comparative Example 7-1)
   ESI-HR MS: Calcd. for [M]3-; 1233.52281. Found; 1233.52225.
- (Sp)-d(ATA(^{p}Cps)6TAT) (nucleic acid oligomer 16, Comparative Example 8-1)
   ESI-HR MS: Calcd. for [M]3-; 1281.52281. Found; 1281.52197.
- (Sp)-d(ATA(Cps)2(^{p}Cps)2(Cps)2TAT) (nucleic acid oligomer 18, Comparative Example 9-1)
   ESI-HR MS: Calcd. for [M]3-; 1230.83527. Found; 1230.83793.

The measurement was performed in a similar manner as in that in Examples 1 to 5 except that the nucleic acid oligomers used in Examples 1 to 5 (nucleic acid oligomers 2, 4, 6, 9, and 10) were changed to a nucleic acid oligomer in Comparative Example 6-1, 7-1, or 9-1 (nucleic acid oligomer 12, 14, or 18), and the double-strand melting temperature (Tm) was obtained. The results are shown in Table 5.

### [Comparative Examples 6-2 and 7-2]

### (Tm Measurement of Phosphate Type Nucleic Acid Oligomer)

The measurement was performed in a similar manner as in that in Examples 1 to 5 except that the nucleic acid oligomers used in Examples from 1 to 5 (nucleic acid oligomers 2, 4, 6, 9, and 10) were changed to d(ATA(C)6TAT) or d(ATA(^{Me}C)6TAT), which is a phosphate type nucleic acid oligomer, and the double-strand melting temperature (Tm) was obtained. The results are shown in Table 5.

**[Table 5]**

| | Kind of target nucleic acid | Nucleic acid oligomer | Number | Substituent at the 5-position | Tm°C | ΔTm°C (Rp-Sp) | ΔTm°C (ps-po) |
|---|---|---|---|---|---|---|---|
| Example 6 | RNA | (Rp)-d(ATA(Cps)6TAT) | 11 | H | 65.9 | 9.2 | -3.3 |
| Comparative Example 6-1 | RNA | (Rp)-d(ATA(CPS)6TAT | 12 | H | 62.6 | | -12.5 |
| Comparative Example 6-2 | RNA | d(ATA(C)6TAT) | | H | 53.4 | | - |
| Example 7 | RNA | (Rp)-d(ATA(^{Me}Cps)6TAT) | 13 | Methyl | 71.7 | 6.6 | 1.1 |
| Comparative Example 7-1 | RNA | (Sp)-d(ATA(^{Me}CPS)6TAT | 14 | Methyl | 72.8 | | -5.5 |
| Comparative Example 7-2 | RNA | d(ATA(^{Me}C)6TAT) | | Methyl | 66.2 | | - |
| Example 9 | RNA | (Rp)-d(ATA(Cps)2(^{P}Cps)2(Cps)2TAT) | 17 | H and propynyl | 73.4 | 6.6 | - |
| Comparative Example 9-1 | RNA | (Sp)-d(ATA(Cps)2(^{P}Cps)2(Cps)2TAT) | 18 | H and propynyl | 66.8 | | - |

In the Rp isomers of phosphorothioate type nucleic acid oligomers in Examples 6, 7, and 9, the f double-strand orming ability was remarkably higher than that in each of the Sp isomers of phosphorothioate type nucleic acid oligomers (Comparative Examples 6-1, 7-1, and 9-1). In addition, the Rp isomers of phosphorothioate type nucleic acid oligomers in Examples 6 and 7 had double-strand forming ability equal to or greater than that of a natural form phosphate bond nucleic acid oligomer.

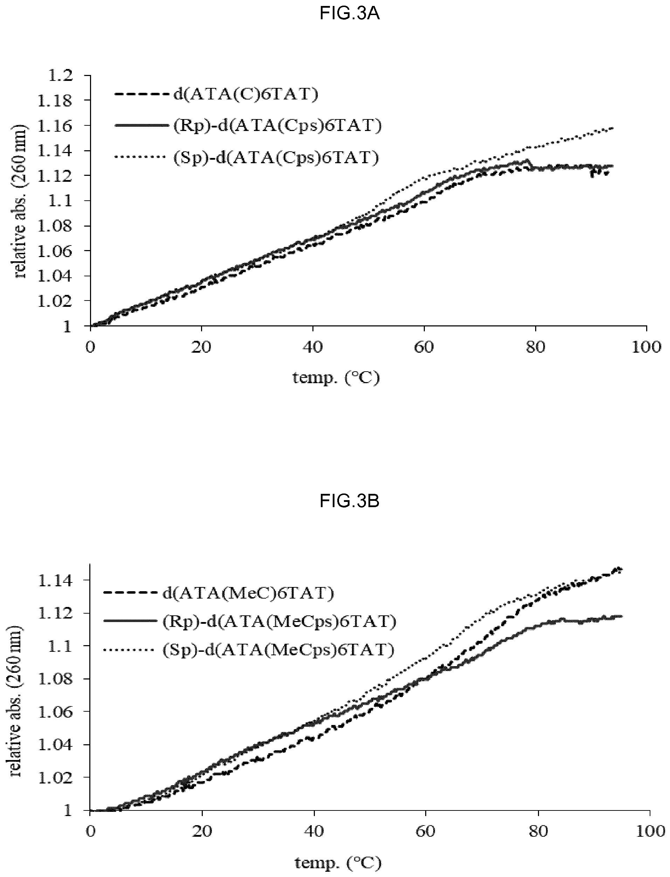

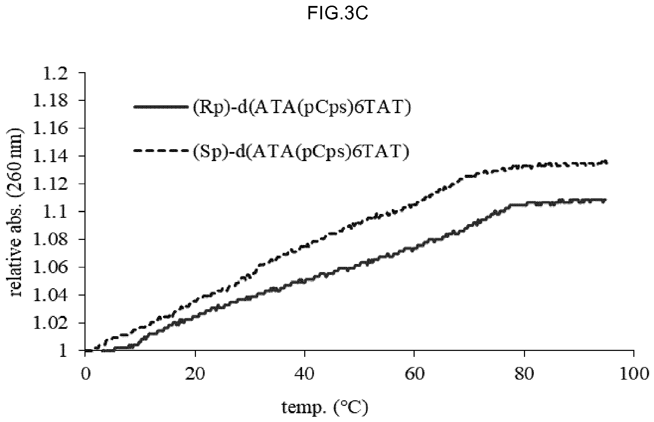

Double-strand melting curves of Example 6, Comparative Example 6-1, and Comparative Example 6-2 are shown in Fig. 3A. Double-strand melting curves of Example 7, Comparative Example 7-1, and Comparative Example 7-2 are shown in Fig. 3B. Double-strand melting curves of Example 9, and Comparative Example 9-1 are shown in Fig. 3C.

As described above, it was indicated that in a phosphorothioate type nucleic acid oligomer for RNA hybrid formation, the absolute steric configuration being Rp results in having a significantly higher Tm value, that is, improving the double-strand forming ability. That is, it was indicated that a nucleic acid oligomer for RNA hybrid formation, which is excellent in double-strand forming ability, can be provided.

The foregoing description of exemplary embodiments of the present invention has been presented for purpose of illustration and description, and it is not intended to be exhaustive or to limit the invention to the precise form disclosed. It is intended that the scope of the invention is defined by the following claims and the equivalents thereof.

### SEQUENCE LISTING

International application under the International Patent Cooperation Treaty CO-F04459-JP17009566_28.app

## Claims

1. A nucleic acid oligomer for RNA hybrid formation, comprising a structure formed of from 3 to 50 continuous units of at least one unit of a nucleotide unit represented by the following General Formula (1) or a nucleotide unit represented by the following General Formula (2), and having a base length of from 8 bases to 50 bases: 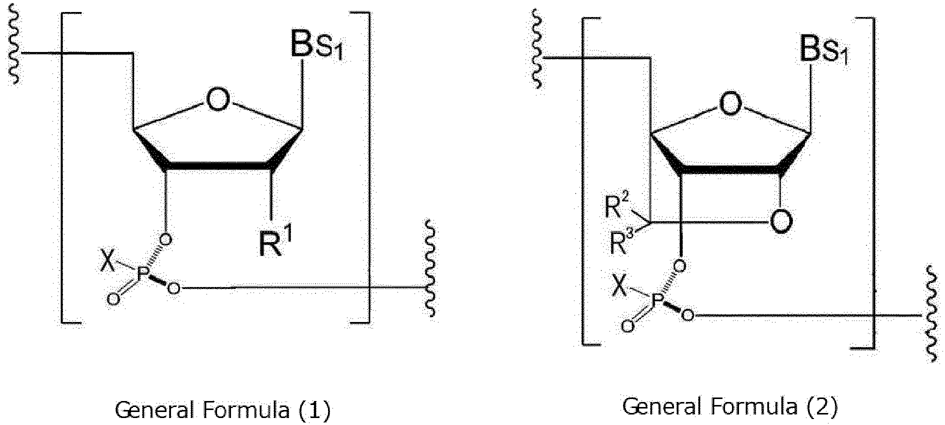 wherein, in General Formula (1), R¹ represents a hydrogen atom, an alkoxy group, an alkenyloxy group, an acyloxy group, a trialkylsilyloxy group, or a halogenyl group; in General Formulae (1) and (2), each Bs₁ independently represents a pyrimidine base that may have a protecting group, and a hydrogen atom bonded to a carbon atom at 5-position of the pyrimidine base is substituted with a propynyl group; and in General Formulae (1) and (2), each X independently represents S⁻Z⁺ or BH₃⁻Z⁺, Z⁺ represents a counter cation, wherein when X is S⁻Z⁺, an absolute steric configuration of the phosphorus atom is Rp, and when X is BH3⁻Z⁺, an absolute steric configuration of the borano group is Sp, and wherein each R² and R³ independently represents a hydrogen atom, or an alkyl group having from 1 to 10 carbon atoms, and R² and R³ may be bonded to each other to form a ring, and wherein, in General Formulae (1) and (2), Bs₁ is a group represented by the following General Formulae (3) or (4): 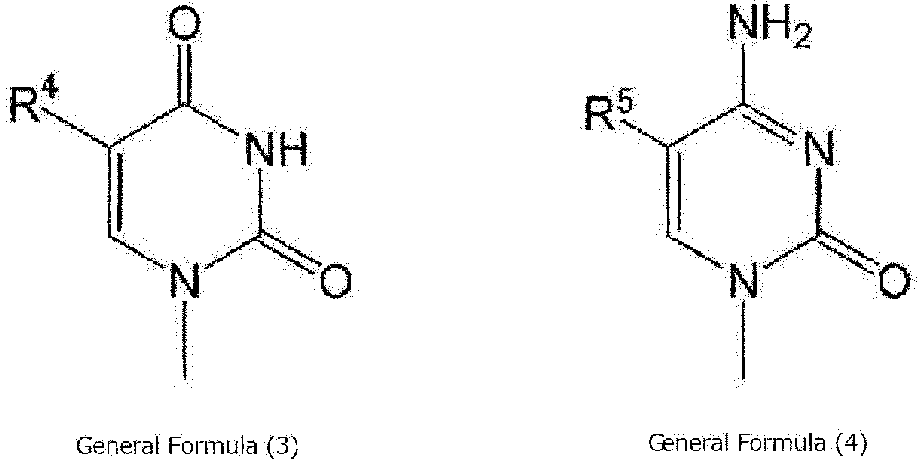 wherein, in General Formula (3), R⁴ represents a propynyl group, and each of a carbonyl group and an amino group in General Formula (3) may have a protecting group; and in General Formula (4), R⁵ represents a propynyl group, and each of a carbonyl group and an amino group in General Formula (4) may have a protecting group.

2. The nucleic acid oligomer for RNA hybrid formation according to claim 1, wherein the nucleic acid oligomer for RNA hybrid formation has a base length of from 10 bases to 30 bases.

3. The nucleic acid oligomer for RNA hybrid formation according to claim 1 or 2, wherein the nucleic acid oligomer for RNA hybrid formation comprises a structure formed of from 5 to 30 continuous units of at least one unit of the nucleotide unit represented by General Formula (1) or the nucleotide unit represented by General Formula (2).

4. An agent for RNA hybrid formation comprising the nucleic acid oligomer for RNA hybrid formation according to any one of claims 1 to 3.

5. A method for forming an RNA hybrid comprising using the nucleic acid oligomer for RNA hybrid formation according to any one of claims 1 to 3.

6. Use of the nucleic acid oligomer for RNA hybrid formation according to any one of claims 1 to 3 as an agent for RNA hybrid formation.

## Patentansprüche

1. Nukleinsäure-Oligomer zur RNA-Hybridbildung, umfassend eine Struktur, welche gebildet ist aus 3 bis 50 kontinuierlichen Einheiten zumindest einer Einheit einer Nukleotideinheit, welche durch die folgende allgemeine Formel (1) dargestellt ist, oder einer Nukleotideinheit, welche durch die folgende allgemeine Formel (2) dargestellt ist, und eine Basenlänge von 8 Basen bis 50 Basen aufweist: 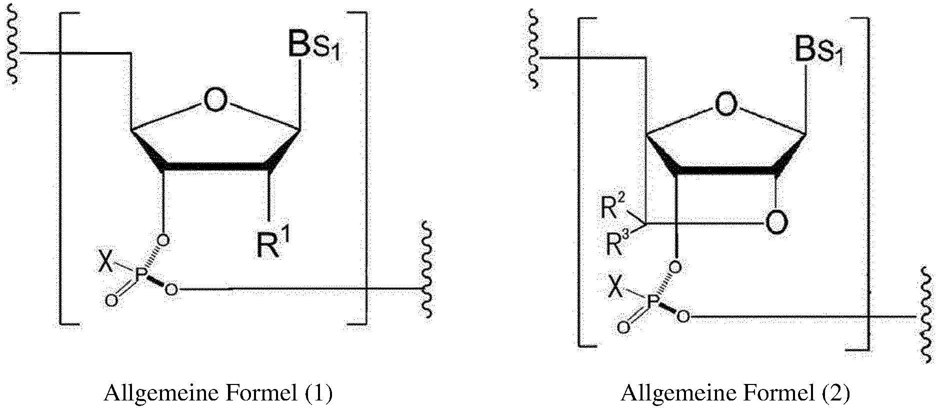 wobei in der allgemeinen Formel (1) R¹ ein Wasserstoffatom, eine Alkoxygruppe, eine Alkenyloxygruppe, eine Acyloxygruppe, eine Trialkylsilyloxygruppe oder eine Halogenylgruppe darstellt; in den allgemeinen Formeln (1) und (2) jedes Bs₁ unabhängig voneinander eine Pyrimidinbase darstellt, die eine Schutzgruppe aufweisen kann, und ein Wasserstoffatom, das an ein Kohlenstoffatom in der 5-Position der Pyrimidinbase gebunden ist, mit einer Propinylgruppe substituiert ist; und in den allgemeinen Formeln (1) und (2) jedes X unabhängig S⁻Z⁺ oder BH₃⁻Z⁺ darstellt, Z⁺ ein Gegenkation darstellt, wobei, wenn X S⁻Z⁺ ist, eine absolute sterische Konfiguration des Phosphoratoms Rp ist, und wenn X BH3⁻Z⁺ ist, eine absolute sterische Konfiguration der Boranogruppe Sp ist, und wobei jedes R² und R³ unabhängig voneinander ein Wasserstoffatom oder eine Alkylgruppe mit 1 bis 10 Kohlenstoffatomen darstellt und R² und R³ miteinander gebunden sein können, um einen Ring zu bilden, und wobei in den allgemeinen Formeln (1) und (2) Bs₁ eine Gruppe ist, die durch die folgenden allgemeinen Formeln (3) oder (4) dargestellt wird: 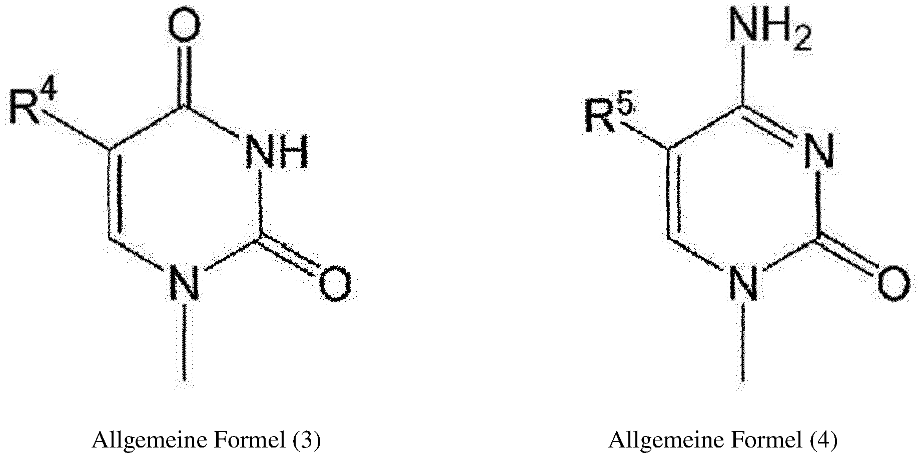 wobei in der allgemeinen Formel (3) R⁴ eine Propinylgruppe darstellt, und sowohl eine Carbonylgruppe als auch eine Aminogruppe in der allgemeinen Formel (3) eine Schutzgruppe aufweisen können; und in der allgemeinen Formel (4) R⁵ eine Propinylgruppe darstellt, und sowohl eine Carbonylgruppe als auch eine Aminogruppe in der allgemeinen Formel (4) eine Schutzgruppe aufweisen können.

2. Nukleinsäure-Oligomer zur RNA-Hybridbildung nach Anspruch 1, wobei das Nukleinsäure-Oligomer zur RNA-Hybridbildung eine Basenlänge von 10 Basen bis 30 Basen aufweist.

3. Nukleinsäure-Oligomer zur RNA-Hybridbildung nach Anspruch 1 oder 2, wobei das Nukleinsäure-Oligomer zur RNA-Hybridbildung eine Struktur umfasst, die aus 5 bis 30 kontinuierlichen Einheiten von mindestens einer Einheit der durch die allgemeine Formel (1) dargestellten Nukleotideinheit oder der durch die allgemeine Formel (2) dargestellten Nukleotideinheit gebildet wird.

4. Mittel zur RNA-Hybridbildung, umfassend das Nukleinsäure-Oligomer zur RNA-Hybridbildung nach einem der Ansprüche 1 bis 3.

5. Verfahren zur Bildung eines RNA-Hybrids, umfassend die Verwendung des Nukleinsäure-Oligomers zur RNA-Hybridbildung nach einem der Ansprüche 1 bis 3.

6. Verwendung des Nukleinsäure-Oligomers zur RNA-Hybridbildung nach einem der Ansprüche 1 bis 3 als Mittel zur RNA-Hybridbildung.

## Revendications

1. Oligomère d'acide nucléique pour formation d'hybride d'ARN, comprenant une structure formée de 3 à 50 unités continues d'au moins une unité d'une unité de nucléotide représentée par la formule générale (1) ou d'une unité de nucléotide représentée par la formule générale (2), et ayant une longueur de bases de 8 bases à 50 bases : 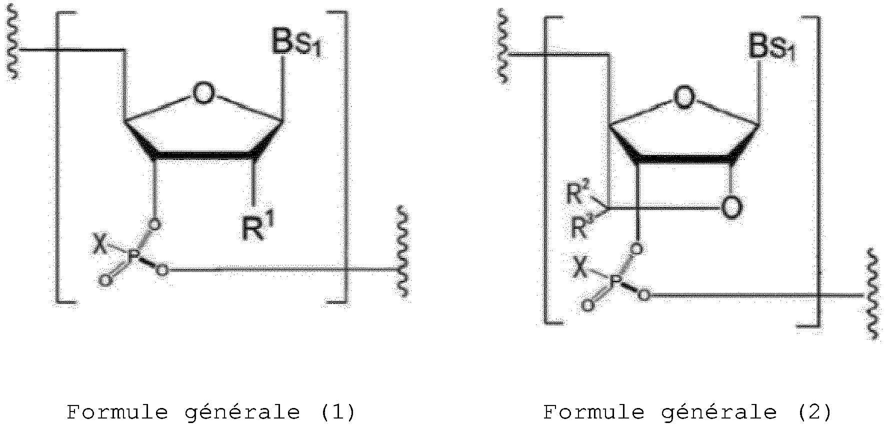 où, dans la formule générale (1), R¹ représente un atome d'hydrogène, un groupe alcoxy, un groupe alcényloxy, un groupe acyloxy, un groupe trialkylsilyloxy ou un groupe halogényle ; dans les formules générales (1) et (2), chaque Bs₁ représente indépendamment une base pyrimidine qui peut avoir un groupe protecteur, et un atome d'hydrogène lié à un atome de carbone en position 5 de la base pyrimidine est substitué avec un groupe propynyle ; et dans les formules générales (1) et (2), chaque X représente indépendamment S⁻Z⁺ ou BH3⁻Z⁺, Z⁺ représente un contre cation, lorsque X est S⁻Z⁺, une configuration stérique absolue de l'atome de phosphore est Rp, et lorsque X est BH₃⁻Z⁺, une configuration stérique absolue du groupe borano est Sp, et chaque R² et R³ représentant indépendamment un atome d'hydrogène, ou un groupe alkyle ayant 1 à 10 atomes de carbone, et R² et R³ peuvent être liés l'un à l'autre pour former un cycle, et, dans les formules générales (1) et (2), Bs₁ étant un groupe représenté par les formules générales (3) ou (4) : 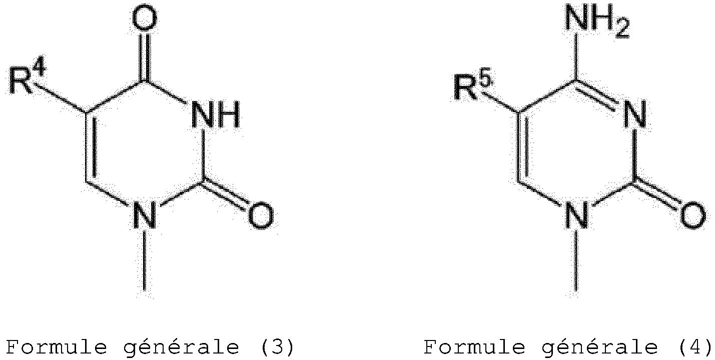 où, dans la formule générale (3), R⁴ représente un groupe propynyle, et chacun d'un groupe carbonyle et d'un groupe amino dans la formule générale (3) peut avoir un groupe protecteur ; et dans la formule générale (4), R⁵ représente un groupe propynyle, et chacun d'un groupe carbonyle et d'un groupe amino dans la formule générale (4) peut avoir un groupe protecteur.

2. Oligomère d'acide nucléique pour formation d'hybride d'ARN selon la revendication 1, ledit oligomère d'acide nucléique pour formation d'hybride d'ARN ayant une longueur de bases de 10 bases à 30 bases.

3. Oligomère d'acide nucléique pour formation d'hybride d'ARN selon la revendication 1 ou 2, ledit oligomère d'acide nucléique pour formation d'hybride d'ARN comprenant une structure formée de 5 à 30 unités continues d'au moins une unité de l'unité de nucléotide représentée par la formule générale (1) ou de l'unité de nucléotide représentée par la formule générale (2).

4. Agent pour formation d'un hybride d'ARN comprenant l'oligomère d'acide nucléique pour formation d'hybride d'ARN selon l'une quelconque des revendications 1 à 3.

5. Procédé de formation d'un hybride d'ARN comprenant l'utilisation de l'oligomère d'acide nucléique pour formation d'hybride d'ARN selon l'une quelconque des revendications 1 à 3.

6. Utilisation d'un oligomère d'acide nucléique pour formation d'hybride d'ARN selon l'une quelconque des revendications 1 à 3 comme agent pour la formation d'un hybride d'ARN.
